# NEUE EUROPÄISCHE PATENTSCHRIFT

(11) **EP 1 129 186 B2**
(45) Veröffentlichungstag und Bekanntmachung des Hinweises auf die Entscheidung über den Einspruch: **30.11.2016**
(45) Hinweis auf die Patenterteilung: 05.09.2007
(21) Anmeldenummer: 99953425.8
(22) Anmeldetag: 10.11.1999
(51) Int. Cl.: C12N 15/12, C07K 14/755, C12P 21/02, A61K 38/37

(54) **EIN FAKTOR VIII-POLYPEPTID MIT FAKTOR VIII:C-AKTIVITÄT**
A FACTOR VIII-POLYPEPTIDE WITH FACTOR VIII:C-ACTIVITY
POLYPEPTIDE DU FACTEUR VIII AVEC ACTIVITE DE FACTEUR VIII:C

(30) Priorität: 10.11.1998 AT 187298
(43) Veröffentlichungstag der Anmeldung: 05.09.2001
(73) Patentinhaber: Stichting Sanquin Bloedvoorziening, 1066 CX Amsterdam (NL)
(72) Erfinder: LENTING, Petrus, Johannes, NL-1035 TW Amsterdam (NL); VAN MOURIK, Jan, Aart, NL-1171 HD Badhoevedorp (NL); MERTENS, Koenraad, NL-2314 ES Leiden (NL); PANNEKOEK, Hans, NL-1431 AR Aalsmeer (NL); TURECEK, Peter, A-3400 Klosterneuburg (AT); SCHWARZ, Hans-Peter, A-1180 Wien (AT); SCHEIFLINGER, Friedrich, A-1090 Wien (AT)
(74) Vertreter: V.O.
(86) Internationale Anmeldenummer: PCT/AT1999/000272
(87) Internationale Veröffentlichungsnummer: WO 2000/028021

(56) Entgegenhaltungen:
- EP-A- 0 808 901
- WO-A-87/07144
- WO-A-97/03195
- US-A- 5 364 771
- PIPE, S.W. AND KAUFMANN, R.J.: "Factor VIII C2 domain missense mutations exhibit defective trafficking of biologically functional proteins" JOURNAL OF BIOLOGICAL CHEMISTRY, Bd. 271, Nr. 41, 1996, Seiten 25671-25676, XP000882876
- LENTING P.J. ET AL: 'The Light Chain of Factor VIII Comprises a Binding Site for Low Density Lipoprotein Receptor-related Protein' THE JOURNAL OF BIOLOGICAL CHEMISTRY Bd. 274, Nr. 34, 1999, Seiten 23734 - 23739
- BOVENSCHEN N. ET AL: 'Low Density Lipoprotein Receptor-related Protein and Factor IXa Share StructuralRequirements for Binding to the A3 Domain of Coagulation Factor VIII*' THE JOURNAL OF BIOLOGICAL CHEMISTRY Bd. 278, Nr. 11, 14 März 2003, Seiten 9370 - 9377

### SEQUENZPROTOKOLL

<110> Baxter AG
<120> Ein Faktor VIII-Polypeptid mit Faktor VIII:C-Aktivität
<130> R 36089
<140> PCT/AT99/00272
   <141> 1999-11-10
<150> A 1872/98
   <151> 1998-11-10
<160> 14
<170> PatentIn Ver. 2.1
<210> 1
   <211> 33
   <212> DNA
   <213> Künstliche Sequenz
<220>
   <223> Beschreibung der künstlichen Sequenz:Primer
<400> 1
   ttaggatcca ccactatgca aatagagctc tcc 33
<210> 2
   <211> 34
   <212> DNA
   <213> Künstliche Sequenz
<220>
   <223> Beschreibung der künstlichen Sequenz:Primer
<400> 2
   agtagtacga gttatttcac taaagcagaa tcgc 34
<210> 3
   <211> 35
   <212> DNA
   <213> Künstliche Sequenz
<220>
   <223> Beschreibung der künstlichen Sequenz:Primer
<400> 3
   ttgcgattct gctttagtga aataactcgt actac 35
<210> 4
   <211> 32
   <212> DNA
   <213> Künstliche Sequenz
<220>
   <223> Beschreibung der künstlichen Sequenz:Primer
<400> 4
   attgcggccg ctcagtagag gtcctgtgcc tc 32
<210> 5
   <211> 33
   <212> DNA
   <213> Künstliche Sequenz
<220>
   <223> Beschreibung der künstlichen Sequenz:Primer
<400> 5
   aatgcggccg cttcaattta aatcacagcc cat 33
<210> 6
   <211> 32
   <212> DNA
   <213> Künstliche Sequenz
<220>
   <223> Beschreibung der künstlichen Sequenz:Oligonucleotid
<400> 6
   ccggagatta ttacgaggac agttatgaag ac 32
<210> 7
   <211> 28
   <212> DNA
   <213> Künstliche Sequenz
<220>
   <223> Beschreibung der künstlichen Sequenz:Oligonucleotid
<400> 7
   gtcttcataa ctgtcctcgt aataatct 28
<210> 8
   <211> 83
   <212> DNA
   <213> Künstliche Sequenz
<220>
   <223> Beschreibung der künstlichen Sequenz:Oligonucleotid
<400> 8
   cgaattcacc cccagatttg ggaacaccag attgccctga ggctggagat tctgggctgc 60
   gaggcacagc agcagtactg agc 83
<210> 9
   <211> 85
   <212> DNA
   <213> Künstliche Sequenz
<220>
   <223> Beschreibung der künstlichen Sequenz:Oligonucleotid
<400> 9
   ggccgctcag tactgctgct gtgcctcgca gcccagaatc tccagcctca gggcaatctg 60
   gtgttcccaa atctgggggt gaatt 85
<210> 10
   <211> 60
   <212> DNA
   <213> Künstliche Sequenz
<220>
   <223> Beschreibung der künstlichen Sequenz:Oligonucleotid
<400> 10
   ctagaaccac cgttagtggc tcgctacgtg cgactgcacc cccagagttg ggctcaccat 60
<210> 11
   <211> 53
   <212> DNA
   <213> Künstliche Sequenz
<220>
   <223> Beschreibung der künstlichen Sequenz:Oligonucleotid
<400> 11
   attgccctga ggctggaggt tctgggctgc gatactcagc agccagcttg agc 53
<210> 12
   <211> 32
   <212> DNA
   <213> Künstliche Sequenz
<220>
   <223> Beschreibung der künstlichen Sequenz:Oligonucleotid
<400> 12
   ggccgctcaa gctggctgct gagtatcgca gc 32
<210> 13
   <211> 50
   <212> DNA
   <213> Künstliche Sequenz
<220>
   <223> Beschreibung der künstlichen Sequenz:Oligonucleotid
<400> 13
   ccagaacctc cagcctcagg gcaatatggt gagcccaact ctgggggtgc 50
<210> 14
   <211> 31
   <212> DNA
   <213> Künstliche Sequenz
<220>
   <223> Beschreibung der künstlichen Sequenz:Oligonucleotid
<400> 14
   agtcgcacgt agcgagccac taacggtggt t 31

## Beschreibung

Die vorliegende Erfindung betrifft modifizierte Faktor VIII (FVIII)-Polypeptide mit FVIII : C-Aktivität, welche Modifikationen in der A3-und/oder C1 -Domäne der Sequenz der leichten Kette von Faktor VIII aufweisen, Nukleinsäuremoleküle, die für solche modifizierte Faktor VIII-Polypeptide codieren, Vektoren und Wirtszellen, die die Nukleinsäuremoleküle enthalten und Zusammensetzungen, die das Faktor-VIII-Polypeptid enthalten, zur Behandlung von Blutungsstörungen.

Die Blutstillung involviert das Zusammenwirken verschiedener hämostatischer Reaktionsrouten, die schließlich zur Thrombus Bildung führen. Thromben sind Ablagerungen von Blutbestandteilen an der Oberfläche der Gefäßwand und bestehen hauptsächlich aus aggregierten Blutplättchen und unlöslichem, vernetztem Fibrin. Die Fibrinbildung findet durch die beschränkte Proteolyse von Fibrinogen durch das Gerinnungsenzym Thrombin statt. Dieses Enzym ist das Endprodukt der Gerinnungskaskade, einer Reihenfolge von Zymogenaktivierungen, die an der Oberfläche aktivierter Blutplättchen und Leukozyten stattfinden, und einer Vielfalt vaskulärer Zellen (für einen Überblick vgl. K. G. Mann et al., Blood, 1990, Bd. 76, S. 1-16).

Eine Schlüsselfunktion in der Gerinnungskaskade liegt in der Aktivierung von Faktor X durch den Komplex aus aktiviertem Faktor IX (Faktor IXa) und aktiviertem Faktor VIII (Faktor VIIIa). Eine Defizienz oder eine Dysfunktion der Bestandteile dieses Komplexes ist mit der als Hämophilie bekannten Blutungskrankheit verbunden (J. E. Sadler & E. W. Davie : Hemophilia A, Hemophilia B, and von Willebrand's disease, in G. Stamatoyannopoulos et al. (Hrsgb.) : The molecular basis of blood diseases. W. B. Saunders Co., Philadelphia, 1987, S. 576-602). Hämophilie A bezieht sich auf eine Defizienz der Faktor VIII-Aktivität, wogegen Hämophilie B sich auf eine Faktor IX-Defizienz bezieht. Die gängige Behandlung besteht in der Ersatztherapie unter Verwendung pharmazeutischer Präparationen, die aus dem normalen Gerinnungsfaktor bestehen. Von diesen Gerinnungsstörungen ist Hämophilie A die häufigere, von der etwa einer unter 10.000 Männer betroffen sind. Die Ersatztherapie bei Hämophilie A umfasst die wiederholte Verabreichung von Präparationen, die normalen Faktor VIII enthalten, durch intravenöse Infusion. Das Intervall zwischen den Infusionen ist durch den Abbau der Faktor VIII-Aktivität im Blutkreislauf gegeben. Die Halbwertszeit der Faktor VIII Aktivität nach der Infusion ist je nach Individuum verschieden und liegt im Bereich von 10 bis 30 Stunden. Somit ist für eine prophylaktische Therapie alle zwei bis drei Tage eine Infusion erforderlich. Dies stellt für das Leben von Hämophilie-Patienten eine schwere Belastung dar, insbesondere wenn durch lokale Narbenbildung infolge des häufigen Nadeleinstichs für die intravenöse Infusion der venöse Zugang schwierig geworden ist. Es wäre besonders vorteilhaft, wenn die Infusionshäufigkeit durch die Verwendung von Faktor VIII mit einer längeren Halbwertszeit verringert werden könnte. Die Halbwertszeit von Faktor VIII kann durch Eingreifen in den Mechanismus des Faktor VIII-Abbaus (Clearance) verlängert werden, beispielsweise durch Verringerung der Affinität von Faktor VIII für Rezeptoren, die bei seiner Clearance eine Rolle spielen, entweder direkt, indem man Faktor VIII an seiner (seinen) Bindungsstelle (n) für die betreffenden Clearance-Rezeptoren modifiziert, oder indirekt, indem man Verbindungen verwendet, die in die Wechselwirkung von Faktor VIII mit diesen Rezeptoren eingreifen. Die Gestaltung solcher Agentien wurde jedoch bisher dadurch behindert, dass man den Faktor VIII-Clearance-Mechanismus, die in diesem Prozess involvierten Zellrezeptoren und die in der Faktor VIII-Rezeptor Wechselwirkung involvierten Molekularstellen nicht kannte.

Auf molekularer Ebene gibt es hinsichtlich des Faktor VIII Clearance-Mechanismus begrenzte Kenntnisse. Das Faktor VIII Protein wird als Einzelketten-Polypeptid mit 2332 Aminosäuren synthetisiert, mit der typischen Domänen-Struktur A1-A2-B-A3-C1 C2 (G. A. Vehar et al., Nature, Bd. 312,1984, S. 337-342 ; J. J. Toole et al., Nature, Bd. 312,1984,342-347). Infolge intrazellulärer endoproteolytischer Verarbeitung tritt Faktor VIII als heterodimerer Komplex aus schwerer und leichter Kette in den Blutkreislauf ein. Die leichte Kette umfasst die Aminosäurereste 1649-2332 und enthält die A3-C1-C2-Domänen. Die schwere Kette enthält die Domänen A1-A2-B (Reste 1-1648) und ist infolge der begrenzten Proteolyse an einer Anzahl von Positionen innerhalb der B-Domäne heterogen. Das Faktor VIII-Heterodimer hat keine biologische Aktivität, wird aber als Cofaktor des Enzyms Faktor IXa nach proteolytischer Aktivierung durch Thrombin oder Faktor Xa aktiv. Die Proteolyse betrifft sowohl die schwere als auch die leichte Kette von Faktor VIII (M. J. S. H. Donath et al., J. Biol. Chem., Bd. 270,1995, S. 3648-3655) und führt zur Abspaltung eines Amino-terminalen Fragments von der leichten Kette, und zu einer Spaltung an den Domänenverbindungsstellen in der schweren Kette (zwischen den Domänen A1-A2 und A2-B). Der aktivierte Cofaktor wird Faktor Villa genannt und ist ein Heterotrimer, bestehend aus der A1-Domäne, der A2-Domäne und der leichten Kette, die die Domänen A3-C1-C2 umfasst.

Es ist auf diesem Gebiet wohlbekannt, dass die Halbwertszeit des nicht-aktivierten Faktor VIII-Heterodimers stark vom Vorhandensein des von Willebrand-Faktors abhängt, der eine starke Affinität für Faktor VIII (aber nicht Faktor Villa) hat und als Träger-Protein dient (J. E. Sadler und E. W. Davie : Hemophilia A, Hemophilia B and von Willebrand's disease, in G. Stamatoyannopoulos et al. (Hrsgb.) : The molecular basis of blood diseases. W. B. Saunders Co., Philadelphia, 1987, S. 576-602). Es ist bekannt, dass Patienten mit von Willebrand-Krankheit des Typs 3, die keinen nachweisbaren von Willebrand-Faktor in ihrem Blutkreislauf aufweisen, auch eine sekundäre Defizienz an Faktor VIII aufweisen. Weiters beträgt die Halbwertszeit von intravenös verabreichtem Faktor VIII bei diesen Patienten 2 bis 4 Stunden, was erheblich kürzer ist als die bei Hämophilie-A-Patienten beobachteten 10 bis 30 Stunden.

Diese Feststellungen implizieren, dass Faktor VIII zu einer raschen Clearance aus dem Blutkreislauf neigt, und dass dieser Prozess in gewissem Ausmaß durch die Komplex-Bildung mit seinem natürlichen Träger, von Willebrand-Faktor, inhibiert wird. Trotzdem bleibt die Halbwertszeit unerwünscht kurz.

Kürzlich wurde in einem vorläufigen Bericht angedeutet, dass durch Thrombin aktivierter Faktor VIII an Low Density Lipoprote in-Rezeptor-Protein ("LRP") bindet (A. Yakhyaev et al., Blood, Bd. 90 (Suppl. 1), 1997,126-I (Abstract). Dieses Abstract beschreibt die Zellaufnahme und den Abbau von Thrombin-aktivierten Faktor VIII-Fragmenten und berichtet, dass die A2-Domäne, jedoch nicht die anderen beiden Untereinheiten des Faktor Vllla-Heterotrimers, mit zellgebundenem LRP in Wechselwirkung tritt. Die Autoren schlagen vor, dass die A2-Domänenbindung an LRP die lose Wechselwirkung der A2-Domäne im Faktor Vllla-Heterotrimer weiter destabilisiert und so die Faktor VIIIa-Aktivität nach unten reguliert.

Man weiß, dass LRP einer der Rezeptoren ist, die bei der Clearance verschiedener Proteine eine Rolle spielen. LRP ist auf diesem Gebiet auch als alpha2-Macroglobulin-Rezeptor bekannt, und er gehört zur Familie der Low Density Lipoprotein (LDL)-Rezeptoren. Er besteht aus zwei nichtkovalent verbundenen Polypeptid-Ketten : einer alpha-Kette (515 kDa) und einer β-Kette (85 kDa) [zwecks Überblicks vgl. D. K. Strickland et al., FASEB J Bd. 9,1995, S. 890-898]. LRP ist ein Multiligand-Rezeptor für Lipoprotein-und-Proteinase-Katabolismus. Die β-Kette enthält eine Transmembran-Domäne und einen kurzen cytoplasmatischen Schwanz, der für die Endocytose essentiell ist. Die alpha-Kette fungiert als große Ektodomäne und enthält drei Typen von Repeats: epidermale Wachstumsfaktor-artige Domänen, Tyr-Trp-Thr Asp-Sequenzen und LDL-Rezeptor-Klasse A-Domänen. Diese Klasse-A Domänen liegen in vier getrennten Cluster vor, den sogenannten Cluster 1 (2 Domänen), II (8 Domänen), III (20 Domänen) und IV (11 Domänen), für welche gezeigt wurde, dass sie bei der Liganden-Bindung eine Rolle spielen. LRP wird in einer Vielzahl von Geweben exprimiert, insbesondere in Plazenta, Lunge, Gehirn und Leber. In der Leber ist LRP auf Parenchymzellen und Kupffer-Zellen vorhanden. Außerdem wird LRP in einer Vielzahl von Zelltypen, wie Fibroblasten, glatten Muskelzellen, Leydig-und Sertoli-Zellen und Monozyten exprimiert. Die Differenzierung von Monozyten zu Makrophagen ist mit einem drastischen Anstieg in der LRP-Expression verbunden. Schließlich wird LRP auch in Zellarten wie Affen-Nierenzellen (COS) oder Chinese Hamster-Ovarien Zellen (CHO) exprimiert (D. J. FitzGerald et al., J. Cell Biol. Bd. 129,1995, S. 1533-1541), die beide häufig für die Expres sion von Säugerproteinen, einschließlich Faktor VIII, verwendet werden (R. J. Kaufman et al., Blood Coag. Fibrinol. Bd. 8 (Suppl. 2), 1997, S. 3-14).

LRP spielt eine Rolle bei der Clearance einer Vielfalt von Liganden, einschließlich Proteasen, Inhibitoren vom Kunitz-Typ, Protease-Serpin-Komplexen, Lipasen und Lipoproteinen, was nahelegt, dass LRP bei verschiedenen physiologischen und pathophysiologischen Clearance-Prozessen eine wesentliche Rolle spielt (Narita et al., Blood, Bd. 2, S. 555-560,1998 ; Orth et al., Proc. Natl. Acad. Sci., Bd. 89, S. 7422-7426,1992 ; Kounnas et al., J. Biol. Chem., Bd. 271, S. 6523-6529,1996). Seine physiologische Bedeutung geht insbesondere aus der Feststellung hervor, da$ LRP-Knock-out-Mäuse das embryonale Stadium nicht überleben (Herz, J. Curr. Opin. Lipidol. Bd. 4,1993, S. 107-113). Die Sekretion von LRP kann dadurch erschwert werden, dass es mit vielfachen Liganden in Wechselwirkung tritt. Innerhalb der Zelle ist LRP jedoch mit seinem Chaperone-Protein, dem Rezeptor-assoziierten Protein (RAP), assoziiert. Wenn es an RAP gebunden ist, kann das LRP mit keinem seiner bekannten Liganden in Wechselwirkung treten (Herz et al., J. Biol. Chem., Bd. 266, S. 2123221238,1991).

Die Wechselwirkung von LRP mit seinen natürlichen Liganden kann durch lösliche LRP-Fragmente wirksam blockiert werden. Diese können mittels vielerlei, auf diesem Gebiet bekannten Methoden erhalten werden, einschließlich rekombinanter Techniken, und sehen als solche einen Zugang zu wirksamen LRP-Antagonisten vor (I. R. Horn, J. Biol. Chem., Bd. 272,1997, S. 13608-13613; B. Vash et al., Blood, Bd. 92,1998, S. 3277-3285).

Angesichts der typischen Rolle von LRP bei der Clearance von Proteasen, Inhibitoren und Protease-Inhibitor-Komplexen, ist festzustellen, dass LRP auch den aktivierten, nicht-enzymatischen Cofaktor Faktor Villa bindet (A. Yakhyaev et al., Blood Bd. 90 (Suppl. 1), 1997,126-I (Abstract)). Während diese Offenbarung eine Rolle von LRP bei der Faktor VIIIa-Regulierung andeutet, fehlt ihr doch jeglicher Hinweis auf eine Rolle bei der LRP-Regulierung von nicht-aktiviertem, heterodimerem Faktor VIII, ob wohl dies für die Clearance von Faktor VIII aus dem Blutkreislauf-und somit für die Halbwertszeit von Faktor VIII-potentiell von Interesse wäre.

Im Stand der Technik wurden mehrere Versuche unternommen, die pharmakokinetischen Profile von Faktor VIII zu verbessern, einschließlich Modifikationen in verschiedenen Regionen der FVIII Polypeptide :

Die WO 87/07144 beschreibt verschiedene Modifikationen an proteolytischen Schnittstellen, die Arginin-und Lysin-Reste umfassen, um die Labilität der Moleküle für eine spezifische Proteasekatalysierte Spaltung, beispielsweise die Faktor Xa Schnittstelle zwischen Arg 1721 und Ala 1722, zu reduzieren.

Die WO 95/18827, WO 95/18828 und WO 95/18829 beschreiben Faktor VIII-Derivate mit Modifikationen im A2-Bereich der schweren Kette.

Die WO 97/03193 offenbart Faktor VIII-Polypeptid-Analoga, bei welchen die Modifikationen die Änderung der Metallbindungseigenschaften des Moleküls umfassen.

In der WO 97/03195 sind Faktor VIII : C-Polypeptid-Analoga beschrieben, bei welchen Modifikationen an einem oder mehreren Aminosäureresten, die an einen Arg-Rest angrenzen, vorgesehen sind.

Die EP-0 808 901 beschreibt die Konstruktion von Faktor VIII Varianten mit mindestens einer Mutation in mindestens einer immundominanten Region von Faktor VIII und die Verwendung dieser Faktor VIII-Variante zur Behandlung von Patienten mit Faktor VIII-Inhibitoren. Diese Modifikationen führen nicht zu einer verlängerten Halbwertszeit oder erhöhten Stabilität der Faktor VIII-Variante, und zwar weder in vivo noch in vitro.

Mutationen im Bereich der Faktor VIII C2-Domäne (R2307Q R2307L, R2305F, und Y2332F) werden von Pipe et al. (JBC, 271 (41) (1996), 25671-25676) beschrieben.

Im Hinblick auf den Stand der Technik offenbart keines der Dokumente irgendeinen Hinweis darauf, dass eine Modifikation in der leichten Kette des Faktor VIII zu einer modifizierten Bindungsaffinität gegenüber einem Zellrezeptor führt und infolgedessen zu einer verringerten Clearance des Faktor VIII-Proteins und zu einer verlängerten Halbwertszeit und erhöhten Stabilität des Faktor VIII.

Es ist daher eine Aufgabe der vorliegenden Erfindung, ein Faktor VIII-Polypeptid mit Faktor VIII : C-Aktivität mit erhöhter Halbwertszeit und/oder erhöhter Stabilität des Faktor VIII-Proteins in vivo und/oder in vitro vorzusehen.

Gemäß dieser Aufgabe sieht die vorliegende Erfindung ein humanes Faktor VIII-Polypeptid mit Faktor VIII : C-Aktivität, welches eine Modifikation, zwischen AS 1784 (Ser) und 1831 (Asp), und/oder AS 2108 (Asp) und 2118 (Asn) aufweist, wobei die Modifikation eine Mutation, Deletion oder Insertion ist, die die Bindungsaffinität zu LRP verringert (alle Aminosäurenummerierungen, die in der vorliegenden Anmeldung in Bezug auf die Faktor VIII-Sequenz vorgenommen werden, beziehen sich auf die Nummerierung von Vehar et al. (Nature, Bd. 312, 1984, S. 337-342).

Vorzugsweise befindet sich die Modifikation in der C1-Domäne zwischen AS 2108 (Asp) und 2118 (Asn). Noch bevorzugter befindet sich die Modifikation zwischen AS 2112 (Trp) und 2115 (Tyr).

Im Rahmen der vorliegenden Erfindung stellte es sich heraus, dass die Inhibierung von LRP durch seinen Antagonisten, RAP, zur Akkumulation der leichten Kette von Faktor VIII im Medium führt. Dies beweist, dass die zelluläre Aufnahme des Faktor VIII-Heterodimers einen LRP-abhängigen Mechanismus einschließt.

Überraschenderweise zeigte es sich, dass eine Modifikation in der leichten Kette des Faktor VIII-Polypeptids zu einem ähnlichen Effekt führt, das heißt, einer erhöhten Halbwertszeit und Stabilität des Faktor VIII-Proteins.

Aufgrund der Modifikation im Faktor VIII-Molekül nimmt die Bindungsaffinität zu LRP ab, und die rasche Clearance des Proteins wird inhibiert. Diese Feststellung eröffnet neue Möglichkeiten für eine verbesserte Behandlung von Gerinnungsstörungen, was bei der Herstellung der Faktor VIII-Zusammensetzungen nötig sein könnte.

Aufgrund der Modifikation im Faktor VIII-Polypeptid kann die Zunahme der in vivo-und in vitro-Halbwertszeit des Faktor VIII Moleküls gemäß der vorliegenden Erfindung mindestens 10%, vorzugsweise 25%, mehr bevorzugt 50%, mehr bevorzugt 90%, im Vergleich zum Wildtyp-Faktor VIII-Protein betragen.

Diese Faktor VIII-Polypeptide oder Faktor VIII-Varianten gemäß der vorliegenden Erfindung üben ihre günstige Wirkung aus, weil sie interaktive Regionen (Exo-Stellen,"Exosites") darstellen, die sich auf den Untereinheiten des Faktor VIII-Heterodimers, insbesondere auf der leichten Kette des Faktors VIII (Domänen A3-C1-C2) befinden. Der Ausdruck "Exo-Stelle" wird hierin im weiten Sinn verwendet und bezieht sich auf relativ hydrophile Teile des Proteins, die vornehmlich zur Oberfläche des Faktor VIII-Moleküls hin gerichtet sind (Kyte und Doolittle, J. Mol. Biol., Bd. 157, S. 105-132,1982).

Obwohl dieses Verfahren gemäß Kyte und Doolittle mit Prinzipien arbeitet, die bereits auf diesem Gebiet anerkannt sind und auf der Faktor VIII-Sequenz basiert, wie sie zuvor veröffentlicht wurde, wurde diesen hydrophilen Exo-Stellen bisher praktisch keine Aufmerksamkeit gewidmet.

Beispielsweise inkludiert die Exo-Stelle bei AS Ser 1784 bis Asp 1831 die Bindungsregion von Faktor IX, die bereits in der Literatur beschrieben wurde (AS 1801 bis 1823, P. J. Lenting et al., J. Biol. Chem., Bd. 271, S. 1935-1940). Dies ist ein klarer Hinweis auf die Relevanz der Hydropathie-Plots, die für die Identifizierung der Exo-Stellen verwendet werden. Der Ausdruck "Bindungsstelle" bezieht sich hierin auf ein typisches Sequenzmuster von Aminosäuren, einschließlich natürlicher und synthetischer Analoga derselben, die die minimalen Erfordernisse für die Bindung von nicht-aktiviertem Faktor VIII an LRP aufweisen.

In einer ersten Gruppe besonders bevorzugter Ausführungsformen der Erfindung weist das Polypeptid eine Modifikation in einer oder mehreren der Exo-Stellen innerhalb der Sequenz des Faktor VIII-Polypeptids auf, vorzugsweise der leichten Kette des Faktors VIII, und mehr bevorzugt der Faktor VIII-A3- und C1-Domäne. Weiters stammen diese Polypeptide vorzugsweise aus der Sequenz von humanem Faktor VIII,. obwohl die Erfindung Bindungsstellen, die auf Exo-Stellen von Faktor VIII jeder beliebigen Säuger-Spezies basieren, umfasst.

Die Modifikation kann beispielsweise durch gerichtete in vitro Mutagenese oder PCR oder andere Methoden der Gentechnik, die zum Stand der Technik gehören, die für die spezifische Veränderung einer DNA-Sequenz für gerichteten Austausch von Aminosäuren geeignet sind, durchgeführt werden (Current Protocols in Molecular Biology, Bd. 1, Kap. 8 (Ausubel et al., Hrsgb., J. Wiley and Sons, 1989 & Supp. 1990-93); Protein Engineering (Oxender & Fox Hrsgb., A. Liss, Inc. 1987)). Diese Modifikation kann eine Mutation, Deletion oder Insertion im Bereich der leichten Kette des Faktors VIII sein.

Die vorliegende Erfindung sieht weiters die Nukleinsäure vor, die für jedes der modifizierten Faktor VIII-Proteine, die von der vorliegenden Erfindung umfasst sind, codiert. Die Nukleinsäure kann DNA oder RNA sein. Die Nukleinsäure ist in einem Expressionsvektor enthalten, der die geeigneten Elemente für die Expression dieser DNA oder RNA vorsieht. Der Expressionsvektor kann beispielsweise in der Transkriptionsrichtung eine transkriptionale Regulierungsregion und eine translationale Initiierungsregion aufweisen, die in einer Wirtszelle funktionell sind, eine für das FVIII-Polynucleotid der vorliegenden Erfindung codierende DNA-Sequenz und translationale und transkriptionale Terminationsregionen, die in dieser Wirtszelle funktionell sind, wobei die Expression dieser Nukleinsequenz durch die Initiierungs-und Terminations-Regionen reguliert wird. Der Expressionsvektor kann auch Elemente für die Replikation dieser DNA oder RNA enthalten. Der Expressionsvektor kann ein DNA-oder ein RNA Vektor sein. Beispiele für DNA-Expressions-Vektoren sind pBPV, pSVL, pRc/CMV, pRc/RSV, myogene Vektorsysteme (WO 93/09236) oder Vektoren, die aus Virussystemen stammen, beispielsweise von Vaccinia-Virus, Adenoviren, Adeno-assoziiertem Virus, Herpesviren, Retroviren oder Baculoviren. Beispiele für RNA-Expressions Vektoren sind Vektoren, die von RNA-Viren stammen, wie Retroviren oder Flaviviren.

Für einige spezifische Anwendungen in der Gentherapie, d. h., wenn die Nukleinsäure an sich in ein Organ eines Säugers injiziert wird, kann die Nukleinsäure, DNA sowie RNA, chemisch modifiziert sein. Die chemischen Modifikationen können Modifikationen sein, die die Nukleinsäure vor Nuklease-Verdau schützen, beispielsweise durch Stabilisieren des Gerüsts oder der Termini.

Der Expressionsvektor, der die Nukleinsäure enthält, die für das modifizierte Faktor VIII-Polypeptid gemäß der vorliegenden Erfindung codiert, kann zur Transformation von Wirtszellen verwendet werden, welche dann dieses Polypeptid erzeugen. Die transformierten Wirtszellen können in einem Zellkultursystem gezüchtet werden, um dieses Polypeptid in vitro zu erzeugen. Die Wirtszellen können das modifizierte Faktor VIII-Polypeptid in das Zellkulturmedium abscheiden, aus welchem es aufgereinigt werden kann. Die Wirtszellen können das modifizierte Faktor VIII-Polypeptid auch im Inneren ihrer Zellwände behalten, und das Hybrid-Protein kann aus den Wirtszellen hergestellt werden.

Die Wirtszellen können Zellen sein, die aus dem Körper eines Säugers stammen, beispielsweise Fibroblasten, Keratinozyten, hematopoietische Zellen, Hepatozyten oder Myoblasten, die in vitro mit einem Expressionsvektorsystem transformiert werden, das eine Nukleinsäure gemäß der vorliegenden Erfindung trägt, und in den Säuger re-implantiert werden. Das durch diese Nukleinsäure codierte Faktor VIII-Polypeptid wird durch diese Zellen in vivo synthetisiert, und sie werden eine gewünschte biologische Aktivität im Säuger zeigen. Gemäß einer spezifischen Ausführungsform ist der Säuger ein menschlicher Patient, der an Hämophilie leidet.

Die für das modifizierte Faktor VIII-Polypeptid gemäß der vorliegenden Erfindung codierende Nukleinsäure kann auch verwendet werden, um transgene Tiere zu schaffen, die diese modifizierten Faktor VIII-Polypeptid-Proteine in vivo exprimieren. Bei einer Ausführungsform dieser spezifischen Anwendung können die transgenen Tiere das Faktor VIII-Polypeptid in endogenen Drüsen, beispielsweise in Brustdrüsen, erzeugen, aus welchen diese Proteine abgeschieden werden. Im Falle von Brustdrüsen können diese Faktor VIII-Proteine in die Milch der Tiere abgeschieden werden, aus welcher diese Proteine hergestellt werden können. Die Tiere können Mäuse, Rinder, Schweine, Ziegen, Schafe, Kaninchen oder irgendein anderes wirtschaftlich nützliches Tier sein.

Der Expressionsvektor, der die Nukleinsäure enthält, die für jedes Faktor VIII-Polypeptid codiert, das von der vorliegenden Erfindung umfasst ist, kann weiters an einen Säuger verabreicht werden, ohne vorherige in vitro-Transformation in Wirtszellen. Der praktische Hintergrund für diese Art von Gentherapie ist in mehreren Patentanmeldungen geoffenbart, beispielsweise in der WO 90/11092. Der diese Nukleinsäure enthaltende Expressionsvektor wird mit einem geeigneten Träger, beispielsweise einer physiologischen Pufferlösung, gemischt und in ein Organ, vorzugsweise einen Skelettmuskel, die Haut oder die Leber eines Säugers, injiziert. Der Säuger ist vorzugsweise ein Mensch und mehr bevorzugt ein Mensch, der an einem genetischen Defekt leidet, am meisten bevorzugt leidet der Mensch an einer Blutgerinnungsstörung. Bei einer besonderen Ausführungsform ist der Säuger ein menschlicher Patient, der an Hämophilie leidet, und die Nukleinsäure, die im Expressionsvektor enthalten ist, codiert für das modifizierte Faktor VIII-Polypeptid, wie beschrieben.

Es ist vorteilhaft, dass das modifizierte Faktor VIII-Protein gemäß der vorliegenden Erfindung eine Faktor VIII-Prokoagulans Aktivität aufweist von mindestens 50%, mehr bevorzugt mindestens 80%, insbesondere mindestens 100%, der Faktor VIII-Prokoagulans Aktivität eines Faktor VIII-Proteins ohne die Modifikation, die zur verringerten Bindungsaffinität gegenüber LRP führt, beispielsweise von einer im Handel erhältlichen Faktor VIII-Präparation, die auf rekombinantem oder plasmatischem Faktor VIII : C basiert.

Die Evaluierung der Faktor VIII-Prokoagulans-Aktivität kann mittels jedes geeigneten Tests durchgeführt werden, insbesondere mittels jener Tests, die bei der Untersuchung von Faktor VIII Proben routinemäßig durchgeführt werden, wie des einstufigen Gerinnungstests, wie beispielsweise in Mikaelsson und Oswaldson, Scand. J. Haematol. Suppl. 33, S. 79-86,1984, beschrieben, oder eines chromogenen Tests, wie Faktor VIII IMMUNOCHROM (Immuno).

Die Faktor VIII-Aktivität kann auch durch Messung der Fähigkeit von Faktor VIII, als Cofaktor für Faktor IXa bei der Umwandlung von Faktor X zu Faktor Xa zu wirken, bestimmt werden, wobei ein chromogenes Substrat für Faktor Xa verwendet wird (Coatest Factor VIII, Chomogenix, Moelndal, Schweden). Zusätzlich können andere Tests, die dazu dienen, die Menge der Faktor VIII-Aktivität in einer Probe zu bestimmen, verwendet werden, um die Faktor VIII-Aktivität der modifizierten Proteine, die in der vorliegenden Erfindung beschrieben sind, zu testen.

Der tatsächliche Test, ob irgendein neu modifiziertes Faktor VIII-Protein einen bestimmten Prozentsatz an Faktor VIII-Prokoagulans-Aktivität aufweist, wird vorzugsweise parallel mit einem Test auf dasselbe Faktor VIII-Molekül ohne die Modifikation in der LRP-Bindungs-Domäne (z. B. Faktor VIII-Wildtyp oder ein voll aktiver Faktor VIII mit deletierter B-Domäne) durchgeführt. Bei einem solchen geeichten Test des mutierten Faktor VIII-Moleküls kann die relative Prokoagulans-Aktivität (der Prozentsatz der Aktivität im Vergleich zu 100% Aktivität des Wildtyps oder des Faktors VIII mit B-Domänen-Deletion) ohne das Risiko eines auf Millieu-Faktoren zurückzuführenden Fehlers untersucht werden. Da bei in vitro-Tests auf Faktor VIII-Prokoagulans-Aktivität die Ergebnisse oft durch Fehler, die auf deren artifizielle Natur zurückzuführen sind, beeinflusst sind, werden beide Eigenschaften vorzugweise auch durch in vivo-oder ex vivo-Tests untersucht, um hinsichtlich der Aktivitätswerte verlässlichere Ergebnisse zu erhalten.

Wie bei den in vitro-Tests, ist auch ein Parallel-Testen des Faktor VIII-Moleküls ohne die Modifikation bevorzugt, wenn man in vivo-Tests durchführt. Geeignete Tiermodelle zur Evaluierung der Faktor VIII : C-Aktivität sind in der WO 95/01570 und der EP 0 747 060 beschrieben.

Die Präparation gemäß der vorliegenden Erfindung kann als pharmazeutische Präparation mit modifiziertem Faktor VIII-Polypeptid gemäß der vorliegenden Erfindung als Einzelkomponenten-Präparation oder in Kombination mit anderen Komponenten, als Mehrfachkomponenten-System vorgesehen sein. Bei einer speziellen Ausführungsform können die Faktor VIII-Proteine oder die modifizierten Faktor VIII-Moleküle gemäß der Erfindung mit einem oder mehreren Polypeptiden kombiniert sein, die die Bindung und Internalisierung von Faktor VIII durch Low Density Lipoprotein-Rezeptor-verwandtes Protein (LRP) selektiv inhibieren, wobei das Polypeptid RAP ist.

Gemäß einem anderen Ziel sieht die vorliegende Erfindung eine Zusammensetzung vor, die ein Faktor VIII-Molekül und ein oder mehrere Polypeptide aufweist, die in die Wechselwirkung zwischen Faktor VIII und LRP antagonistisch eingreifen und daher die Bindung und Internalisierung von Faktor VIII durch Low Density Lipoprotein-Rezeptor-verwandtes Protein (LRP) selektiv inhibieren. Vorzugsweise ist dieses Polypeptid RAP oder ein lösliches LRP-Fragment mit einer antagonistischen Wirkung. Vorzugsweise bindet das lösliche LRP-Fragment an den FVIII in der FVIII-LRP Bindungsregion.

Diese Präparationen können als aktive Komponente der pharmazeutischen Zusammensetzungen zur Behandlung von Patienten, die an genetischen Störungen, vorzugsweise an Gerinnungsstörungen, und am meisten bevorzugt an Hämophilie, beispielsweise an Hämophilie A, leiden, verwendet werden. Diese Verbindungen können weiters als aktive Komponente von pharmazeutischen Zusammensetzungen zur Behandlung von Patienten, die an vorübergehenden Störungen ihrer thrombotischen oder fibrinolytischen Systeme leiden, beispielsweise vor, während oder nach einer Operation, verwendet werden.

Gemäß der vorliegenden Erfindung ist eine pharmazeutische Zusammensetzung zur Verabreichung an Säuger, vorzugsweise Menschen, bestimmt. Bei der Herstellung des pharmazeutischen Produkts, werden die Verbindungen der vorliegenden Erfindung, das modifizierte Faktor VIII-Polypeptid, die Nukleinsäuren, die für dieses codieren oder die transformierten Zellen, die es in vivo exprimieren können, mit physiologisch akzeptablen Trägern gemischt.

Die in der vorliegenden Erfindung geoffenbarten Zusammensetzungen können zur Verabreichung auf jedem geeigneten Weg formuliert sein, und die Erfindung umfasst auch pharmazeutische Zusammensetzungen, die eine therapeutisch wirksame Menge an Faktor VIII enthalten. Solche Zusammensetzungen können auf herkömmliche Weise unter Verwendung eines oder mehrerer pharmazeutisch akzeptabler Träger oder Exzipienten formuliert werden. Geeignete Träger umfassen Verdünnungsmittel oder Füllstoffe, sterile wässerige Medien und verschiedene nicht-toxische organische Lösungsmittel, sind jedoch nicht auf diese beschränkt. Die Zusammensetzungen können in Form von Pulvern, wässerigen Suspensionen oder Lösungen, injizierbaren Lösungen u. dgl. formuliert werden. Geeignete Dosierungsformen können vom Fachmann leicht identifiziert werden.

Das Verfahren gemäß der vorliegenden Erfindung zur Behandlung von Gerinnungsstörungen sollte unter Verwendung eines Dosierungsschemas durchgeführt werden, welches eine maximale therapeutische Reaktion garantiert, bis eine Besserung erreicht ist, und danach der wirksamen Mindestmenge, die einen geeigneten Schutz gegen Blutung bietet. Die Dosierung für die intravenöse Verabreichung kann zwischen etwa 10 und 300 IE/kg Körpergewicht variieren, vorzugsweise zwischen etwa 10 und 100 IE/kg Körpergewicht, und mehr bevorzugt zwischen 20 und 40 IE/kg Körpergewicht. Die geeignete Dosierung kann auch von der allgemeinen Gesundheit des Patienten, dem Alter und anderen Faktoren abhängig sein, die die Reaktion auf das Arzneimittel beeinflussen können. Das Arzneimittel kann durch kontinuierliche Infusion oder in regelmäßigen Intervallen verabreicht werden, um die therapeutische Wirkung auf dem gewünschten Niveau zu halten.

Ein anderer Aspekt der Erfindung betrifft ein Verfahren zur Herstellung von modifizierten Faktor VIII-Molekülen gemäß der Erfindung, die eine Modifikation in der leichten Kette aufweisen. Die für das modifizierte Faktor VIII-Molekül codierende Sequenz wird in ein geeignetes Expressionssystem inseriert, beispielsweise einen Expressionsvektor, und geeignete Zellen werden mit der rekombinanten DNA transfiziert. Vorzugsweise werden permanente Ziellinien etabliert, die den modifizierten Faktor VIII exprimieren. Die Zellen werden unter für die Genexpression optimalen Bedingungen gezüchtet, und modifizierter Faktor VIII wird entweder aus einem Zellkulturextrakt oder aus dem Zellkulturüberstand isoliert. Das rekombinante Molekül kann mittels aller bekannter chromatographischer Methoden weiter gereinigt werden, wie Anionen-oder Kationenaustausch-, Affinitäts-oder Immunoaffinitätschromatographie oder einer Kombination davon.

Der modifizierte Faktor VIII wird vorzugsweise durch rekombinante Expression erzeugt. Sie können auf gentechnischem Weg mit jeglichen üblichen Expressionssystemen hergestellt werden, wie beispielsweise permanenten Zelllinien oder viralen Expressionssystemen. Permanente Ziellinien werden hergestellt durch stabile Integration der Fremd-DNA in das Wirtszellen-Genom von beispielsweise Vero-, MRC5-, CHO-, BHK-, 293-, Sk-Hep1-, insbesondere Leber-und Nierenzellen, Fibroblasten, Keratinozyten oder Myoblasten, Hepatozyten oder Stammzellen (ausgenommen menschliche embryonale Stammzellen), beispielsweise hematopoietischen Stammzellen, oder durch einen episomalen Vektor, der beispielsweise vom Papilloma-Virus stammt. Virus-Expressionssysteme, wie beispielsweise das Vaccinia-Virus-, Baculovirus-oder Retrovirus-System können ebenfalls verwendet werden. Als Zelllinien werden im allgemeinen Vero-, MRC5-CHO-, BHK-, 293-, Sk Hep1-, Drüsen-, Leber-und Nierenzellen verwendet. Als eukaryontische Expressionssysteme können Hefe-, endogene Drüsen- (z. B. Drüsen transgener Tiere) und auch andere Arten von Zellen verwendet werden. Natürlich können auch transgene Tiere für die Expression der erfindungsgemäßen Polypeptide oder Derivate davon verwendet werden. Für die Expression von rekombinanten Proteinen erwiesen sich CHO-DHFR-Zellen als besonders geeignet (Urlaub et al., Proc. Natl. Acad. Sci., USA, Bd. 77, S. 4216-4220,1980).

Für die rekombinante Herstellung von modifiziertem Faktor VIII gemäß der vorliegenden Erfindung können auch prokaryontische Expressionssysteme verwendet werden. Systeme, die eine Expression in E. coli oder B. subtilis ermöglichen, sind besonders geeignet.

Das Faktor VIII-Polypeptid gemäß der vorliegenden Erfindung wird in den jeweiligen Expressionssystemen unter der Kontrolle eines geeigneten Promotors exprimiert. Für die Expression in Eukaryonten sind alle bekannten Promotoren geeignet, wie SV40, CMV, RSV, HSV, EBV, β-Actin, hGH oder induzierbare Promotoren, wie beispielsweise hsp oder Metallothionein-Promotor.

Gemäß der vorliegenden Erfindung kann eine Faktor VIII-cDNA der gesamten Länge sowie jegliche Derivate davon, die Faktor VIII : C Aktivität aufweisen (beispielsweise B-Domänen-deletierte Faktor VIII-Mutanten, FVIII-Mutanten, mit teilweise deletierten B-Domänen) als Ausgangsmaterial für die Konstruktion des modifizierten Faktor VIII-Polypeptids verwendet werden. Es kann von jeder Säuger-Spezies abstammen, vorzugsweise von Human-, Schweine-oder Rinder-Quellen.

Die Erfindung ist in den nachfolgend beschriebenen Beispielen veranschaulicht. Obgleich beispielhaft für die vorliegende Erfindung in Bezug auf die Identifizierung, Herstellung und Verwendung verbesserter Zusammensetzungen mit verringerter Bindung der leichten Kette von Faktor VIII an LRP, soll die Erfindung so ausgelegt werden, dass sie auch auf die LRP-Bindung der schweren Kette von Faktor VIII anwendbar ist. Variationen innerhalb des Blickfeldes des Fachmannes sollen als in den Bereich der vorliegenden Erfindung fallend angesehen werden. Sofern nicht anders definiert, haben alle hierin verwendeten technischen und wissenschaftlichen Ausdrücke dieselbe Bedeutung, wie sie allgemein vom Durchschnittsfachmann auf dem Gebiet, zu dem diese Erfindung gehört, verstanden werden. Obwohl alle Methoden und Materialien, die den hierin beschriebenen ähnlich sind oder äquivalent zu diesen sind, in der Praxis oder zum Testen der vorliegenden Erfindung verwendet werden können, werden die bevorzugten Methoden und Materialien nun beschrieben.

Die folgenden Beispiele und die Figuren veranschaulichen die vorliegende Erfindung, sie begrenzen jedoch in keiner Weise den Umfang der Erfindung.

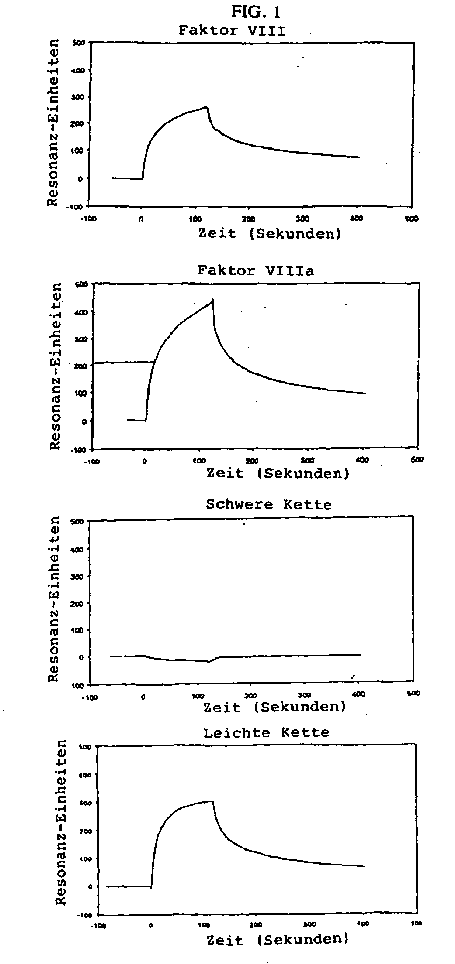

Fig. 1 zeigt die Wechselwirkung zwischen Faktor VIII (Tafel A), Thrombin-aktiviertem Faktor VIII (Faktor VIIIa, Tafel B), der schweren Kette von Faktor VIII (Tafel C) oder der leichten Kette von Faktor VIII (Tafel D) und immobilisiertem LRP unter Verwendung von Oberflächen-Plasmon-Resonanz-Analyse. Details sind in Beispiel I vorgesehen. Ein Vergleich der Tafeln A bis D zeigt, dass Faktor VIII, Thrombin-aktivierter Faktor VIII und die leichte Kette von Faktor VIII, jedoch nicht die schwere Kette von Faktor VIII, effizient mit LRP in Wechselwirkung treten.

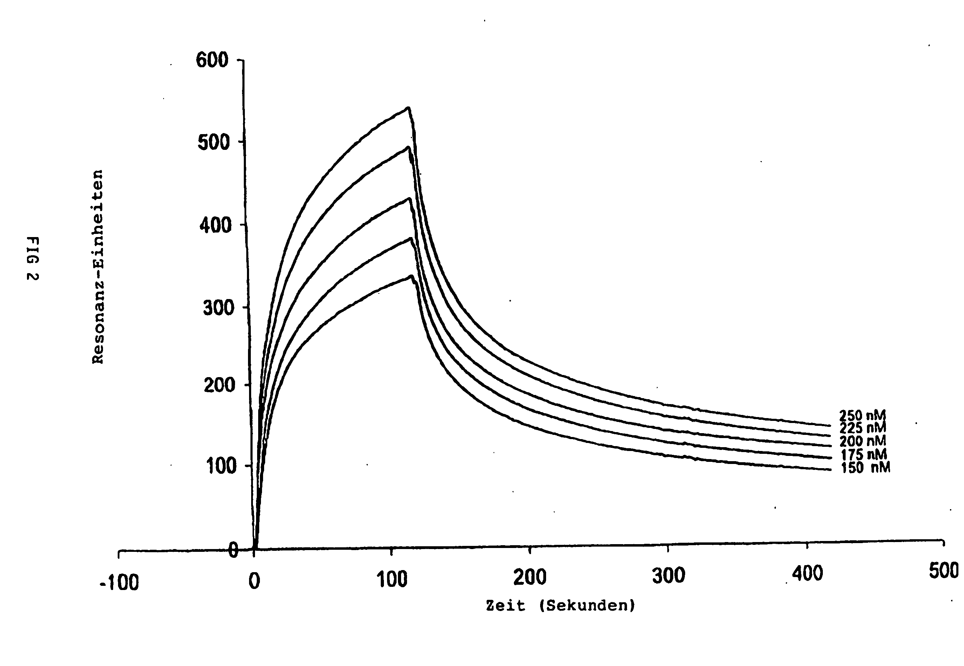

Fig. 2 zeigt, dass die leichte Kette von Faktor VIII auf reversible und Dosis-abhängige Weise an immobilisiertes LRP bindet. Die kinetischen Parameter dieser Wechselwirkung sind in Tabelle II zusammengefasst, die in Beispiel II dargelegt ist. Die Bindung wurde, wie in Beispiel II beschrieben, beurteilt.

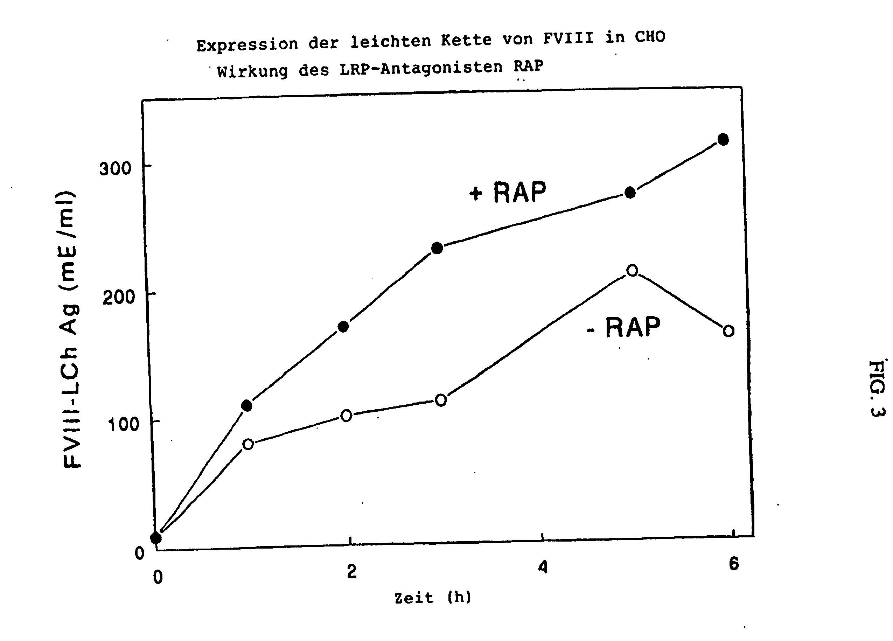

Fig. 3 zeigt die Wirkung des LRP-Antagonisten RAP auf die Konzentration der leichten Kette von Faktor VIII in einem Medium von die leichte Kette von Faktor VIII exprimierenden Zellen. Die Versuche wurden durchgeführt, wie in Beispiel III beschrieben. In Abwesenheit von RAP (offene Symbole) ist der Anstieg der leichten Kette von Faktor VIII im Medium geringer als in seiner Gegenwart (geschlossene Symbole).

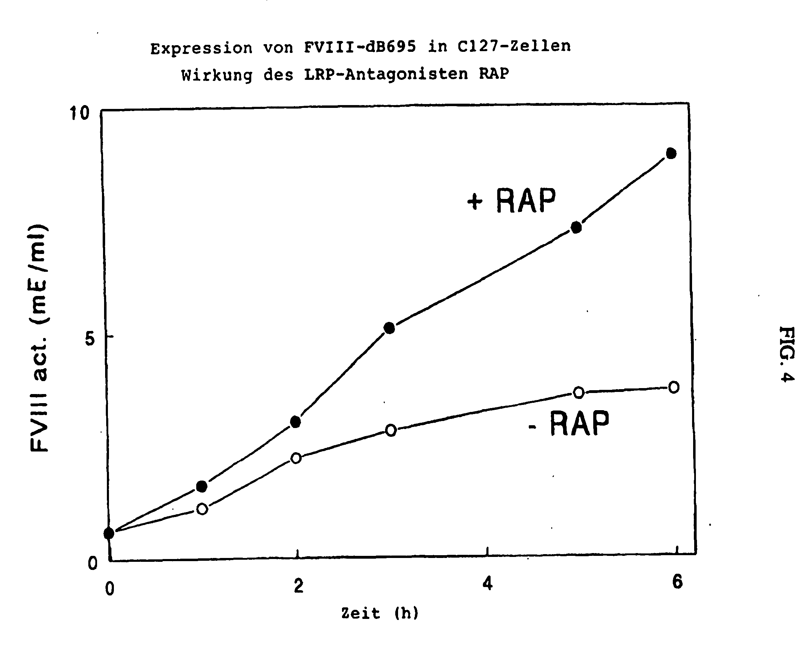

Fig. 4 zeigt die Wirkung des LRP-Antagonisten RAP auf die Konzentration des intakten Faktor VIII-Heterodimers im Medium der Faktor VIII-exprimierenden Zellen. Die Details des Versuchs sind in Beispiel IV beschrieben. In Abwesenheit von RAP (offene Symbole) ist der Anstieg der Faktor VIII-Aktivität geringer als in seiner Anwesenheit (geschlossene Symbole).

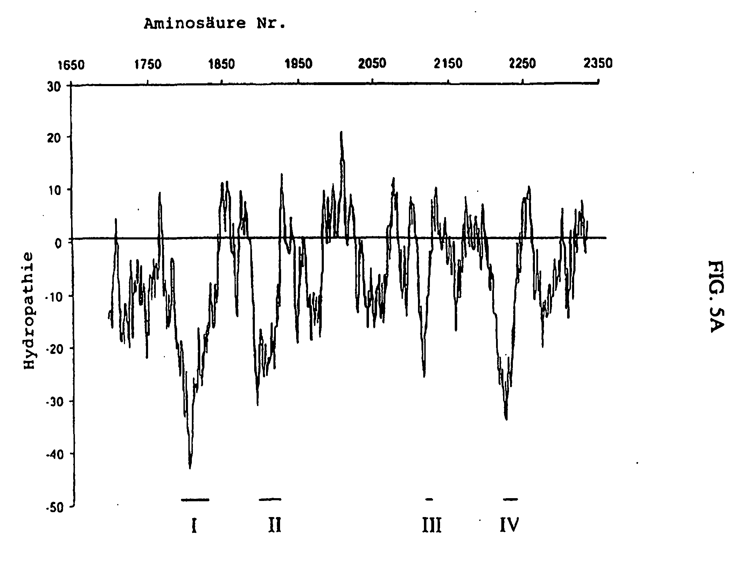

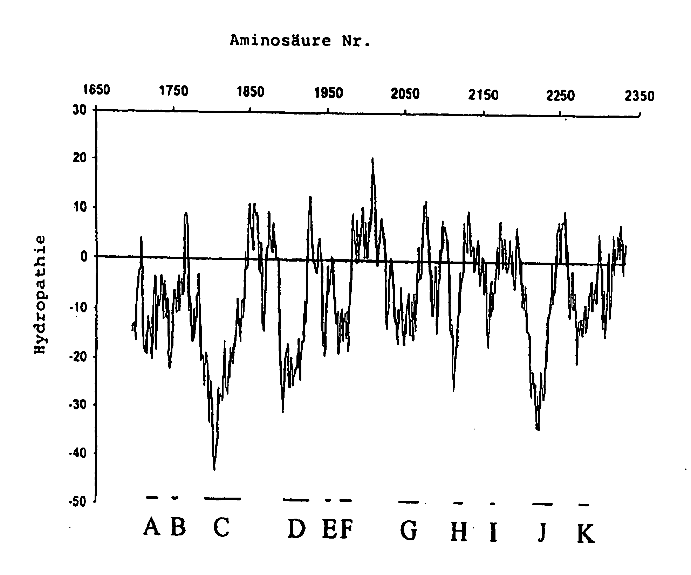

Fig. 5A und B zeigen Hydropathie-Plots der Domänen A3, C1 und C2 der leichten Kette von Faktor VIII. Der Plot wurde wie in Beispiel VI beschrieben erstellt. Der Plot zeigt das Vorhandensein verschiedener einzelner Regionen mit niedrigen Hydropathie-Werten, welche das hydrophile Wesen potentiell exponierter Exo Stellen zeigen. Diese sind als A bis K (Fig. 5A) und I bis IV (Fig. 5B) gezeigt.

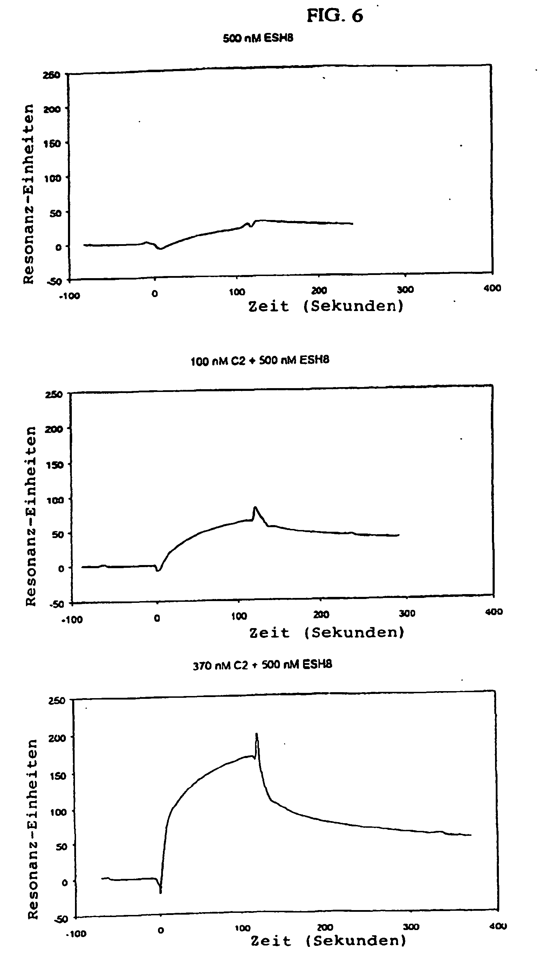

Fig. 6 zeigt die Wechselwirkung der C2-Domäne von Faktor VIII mit immobilisiertem LRP in Anwesenheit des C2-Domänen-gerichteten Antikörpers ESH-8. Die Bindung wurde unter Verwendung von Oberflächen-Plasmon-Resonanz, wie in Beispiel VII beschrieben, analysiert. In Abwesenheit der C2-Domäne zeigt ESH-8 keine signifikante Bindung an immobilisiertes LRP. In Anwesenheit der C2 Domäne wird jedoch eine Dosis-abhängige Zunahme der Bindung an LRP beobachtet. Dies zeigt, dass die C2-Domäne von Faktor VIII an LRP bindet.

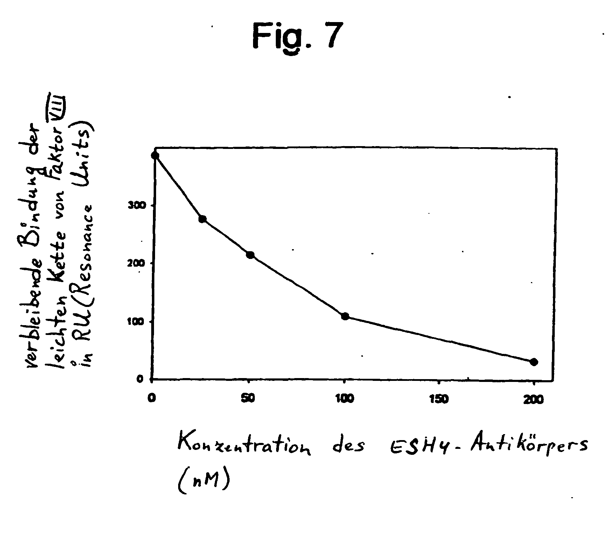

Fig. 7 zeigt die Bindung der Faktor VIII-C2-Domäne an LRP in Gegenwart von ESH4.

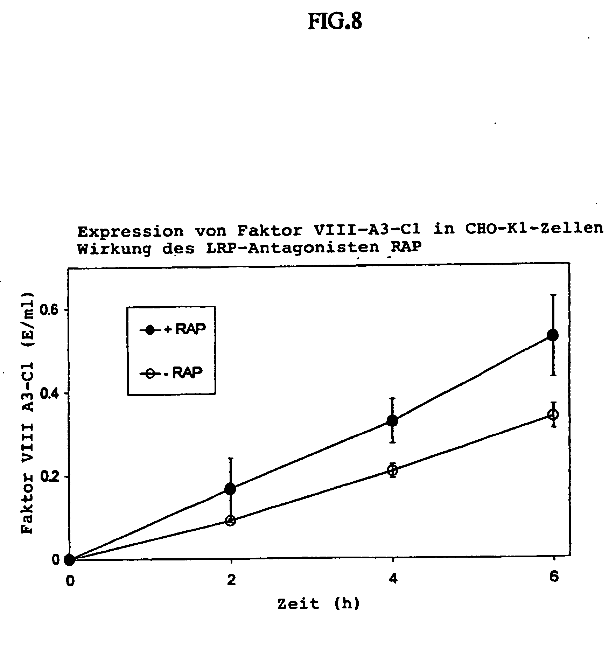

Fig. 8 zeigt den Effekt von RAP auf die Expression von Faktor VIII-A3-C1 in CHO-K1-Zellen.

### Beispiele

### Beispiel I : Die leichte Kette von Faktor VIII hat eine LRP-Bindungsstelle

Die Bindung von Faktor VIII und Untereinheiten desselben an Low Density Lipoprotein-Rezeptor-verwandtes Protein (LRP) wurde unter Verwendung gereinigter Komponenten untersucht. LRP, Faktor VIII, die leichte Kette von Faktor VIII, die schwere Kette von Faktor VIII, Thrombin-aktivierter Faktor VIII wurden unter Verwendung von bereits etablierten Verfahren erhalten (Moestrup S. K. et al., J. Biol. Chem., Bd. 266,1991, S. 14011-1401; Lenting P. J. et al., J. Biol. Chem., Bd. 269,1994, S. 71507155 ; bzw. Curtis J. E. et al., J. Biol. Chem., Bd. 269,1994, S. 6246-6250).

Die Wechselwirkung mit LRP wurde unter Verwendung der Oberflächen-Plasmon-Resonanz (SPR)-Analyse auf einem BIAcoreTM2000 Biosensor-System (Pharmacia Biosensor AB, Uppsala, Schweden) untersucht. LRP wurde in einer Konzentration von 8,3 fmol/mm² auf einem CM5-Sensor-Chip immobilisiert, wobei das Amin-Kopplungsset gemäß den Instruktionen der Hersteller (Pharmacia Biosensor, Uppsala, Schweden) verwendet wurde. Ein Kontroll-Kanal am Sensor-Chip wurde aktiviert und blockiert, wobei Aminkupplungsreagenzien ohne Immobilisierung von Protein verwendet wurden.

Faktor VIII oder Derivate davon wurden in einer Konzentration von 100 nM aber den Kontroll-Kanal geleitet, um die unspezifische Bindung zu bewerten, und aber den LRP-beschichteten Kanal in 50 mM HEPES (pH 7,4), 150 mM NaCl, 2 mM CaCl₂ und 0,005% (V/V) Tween-20 mit einer Strömung von 5 ml/min für einen Zeitraum von 2 min bei 25 C. Die Assoziation zwischen den verschiedenen Proteinen und LRP ist in Fig. 1 gezeigt und in Resonanz Einheiten ausgedrückt. In Tabelle I ist der maximale Anstieg der Resonanz-Einheiten für die verschiedenen Derivate zusammengefasst. Diese Daten zeigen, dass Faktor VIII, Thrombin-aktivierter Faktor VIII und die leichte Kette von Faktor VIII mit LRP in Wechselwirkung treten können. Dagegen konnte die schwere Kette von Faktor VIII LRP nicht binden. Es ist daher offensichtlich, dass der Bindungsanteil von Faktor VIII oder von Thrombin-aktiviertem Faktor VIII für LRP in der A3-C1-C2-Region (Reste 16902232) liegt.

**Tabelle I : Bindung von Faktor VIII und seinen Untereinheiten an immobilisiertes LRP, wie unter Verwendung von SPR-Analyse nachgewiesen. Die Bindung an LRP ist als Resonanz-Einheiten ausgedrückt und ist im Hinblick auf unspezifische Bindung korrigiert.**

| Protein | Bindung (Resonanz-Einheiten) |
|---|---|
| Faktor VIII | 262 |
| schwere Kette von Faktor VIII | 0 |
| leichte Kette von Faktor VIII | 305 |
| Thrombin-aktivierter Faktor VIII | 446 |

### Beispiel II : Assoziationskinetik von immobilisiertem LRP und der leichten Kette von Faktor VIII.

Die kinetischen Parameter für die Wechselwirkung zwischen der leichten Kette von Faktor VIII und immobilisiertem LRP wurden unter Verwendung der SPR-Analyse auf einem BIAcoreTM2000-Biosensor-System (Pharmacia Biosensor AB, Uppsala, Schweden) bestimmt. Dieses Verfahren ist auf diesem Gebiet allgemein bekannt und wurde beispielsweise für die kinetische Analyse der Wechselwirkung zwischen LRP und Rezeptor-assoziiertem Protein (RAP) angewandt (Horn I, in LRPligand interactions : kinetics and structural requirements ; pH. D.-Dissertation, 1997, S. 65-106, Universität Amsterdam). LRP wurde mit einer Konzentration von 6,7 fmol/mm² auf drei Kanälen eines CM5-Sensor-Chips immobilisiert, wie in Beispiel I beschrieben. Ein Kontroll-Kanal zur Bewertung unspezifischer Bindung wurde, wie in Beispiel I beschrieben, bereitet. Verschiedene Konzentrationen der leichten Kette von Faktor VIII (150,175,200,225 und 250 nM) wurden aber den Kontroll-Kanal und aber die mit LRP beschichteten Kanäle in 50 mM HEPES (pH 7,4), 150 mM NaCl, 2 mM CaCl₂ und 0,005 (V/V) Tween 20 mit einer Strömung von 20 ml/min für eine Zeitdauer von 2 min bei 25°C geleitet, damit die Assoziierung stattfinden kann. Danach wurden die Kanäle mit demselben Puffer bei einer ähnlichen Strömung inkubiert, damit die Dissoziierung stattfinden kann. Wie in Fig. 2 gezeigt, wird eine Dosis-abhängige Assoziierungs-und Dissoziierungskurve beobachtet.

Die Daten wurden mittels Biacore Evaluation Software (Pharmacia Biosensor, Uppsala, Schweden) analysiert. Die Datenanalyse zeigte, dass die Wechselwirkung zwischen der leichten Kette von Faktor VIII mit zwei Klassen von Bindungsstellen die beste Übereinstimmung ergab. Danach wurden die Assoziierungs-und Dissozi ierungsgeschwindigkeitskonstanten (kon bzw. koff) für beide Bindungsstellen berechnet. Diese Geschwindigkeitskonstanten wurden nachfolgend verwendet, um die Affinitätskonstanten (Kd) für diese Wechselwirkungen zu erhalten.

**Tabelle II : Geschwindigkeitskonstanten für die Wechselwirkung zwischen der leichten Kette von Faktor VIII und immobilisiertem LRP. Die Datenanalyse weist auf die Wechselwirkung der leichten Kette von Faktor VIII mit zwei Klassen von Bindungsstellen hin, die als A bzw. B repräsentiert sind.**

| Klasse | *kₒₙ* (M⁻¹ s⁻¹) | *k_{off}* (s^{*-*1}) | *K_{d}* (= *k_{off}*/*kₒₙ*; nM) |
|---|---|---|---|
| A | 3,0 x 10⁵ | 5,5 x 10⁻² | 182 |
| B | 7,2 x 10⁴ | 2,7 x 10⁻³ | 37 |

### Beispiel III : Wechselwirkung zwischen der leichten Kette von Faktor VIII und zellgebundenem LRP

Da die leichte Kette von Faktor VIII effizient an LRP in einem System, bei welchem gereinigte Komponenten verwendet werden, bindet, haben wir die Wechselwirkung zwischen der leichten Kette von Faktor VIII und LRP, das an der Oberfläche lebender Zellen exprimiert wird, untersucht. Um die leichte Kette von Faktor VIII zu exprimieren, d. h. die Reste 1649 bis 2332 (Toole J. J. et al., Nature, Bd. 312,1984, S. 342-347), wurde ein Konstrukt hergestellt, das für das Faktor VIII-Signal-Peptid, das mit den Resten 1649 bis 2332 fusioniert ist, codiert. Dieses Konstrukt wurde wie folgt hergestellt. Das früher beschriebene Plasmid pBPV-Faktor VIII-dB695 (K. Mertens et al., Brit. J. Haematol., Bd. 85,1993, S. 133-142) wurde als Vorlage für die Herstellung von zwei Faktor VIII-Fragmenten benützt, wobei die Polymerase Kettenreaktion (PCR) verwendet wurde. Ein Fragment wurde hergestellt, indem der Sense-Primer A1 (5'-TTA GGA TCC ACC ACT ATG CAA ATA GAG CTC TCC-3'), der eine BamH1-Erkennungsstelle und einen Teil enthielt, der für die N-terminalen Reste des Faktor VIII-Signal-Peptids codierte, und der Anti-Sense-Primer A-1 (5' AGT AGT ACG AGT TAT TTC ACT AAA GCA GAA TCG C-3'), der für C-terminale Reste des Faktor VIII-Signal-Peptids und N-terminale Reste der leichten Kette von Faktor VIII codierte, verwendet wurden. Ein zweites Fragment wurde hergestellt, indem der Sense Primer B1 (5'-TTG CGA TTC TGC TTT AGT GAA ATA ACT CGT ACT AC3'), der für die C-terminalen Reste des Faktor VIII-Signal-Peptids und die N-terminalen Reste der leichten Kette von Faktor VIII codierte, und der Anti-Sense-Primer B-1 (5'-ATT GCG GCC GCT CAG TAG AGG TCC TGT GCC TC-3'), der eine Notl-Erkennungsstelle, ein Stop-Codon und einen Teil, der für die C-terminalen Reste der leichten Kette von Faktor VIII codierte, verwendet wurden. In einer zweiten Reaktion wurden die Produkte der beiden Reaktionen als Vorlage für die Konstruktion des resultierenden Fragments, als Faktor VIII-SPLC bezeichnet, benützt, wobei die Primer A1 und B-1 verwendet wurden. Faktor VIII-SPLC bestand aus einer BamHI-Erkennungsstelle, einem für das Faktor VIII-Signalpeptid codierenden Teil, der mit einem für die leichte Kette von Faktor VIII codierenden Teil fusioniert war, einem Stop-Codon und einer Notl-Erkennungsstelle. Faktor VIII-SPLC wurde danach mit BamHI und Notl verdaut und in den Expressionsvektor pcDNA3,1 (Invitrogen, Leek, Niederlande) ligiert, der unter Verwendung derselben Restriktionsenzyme verdaut wurde. Der resultierende Vektor mit der Bezeichnung pcFaktor VIII-LC wurde in Chinese Hamster-Ovarien-K1 (CHO-K1)-Zellen transfiziert (ATCC CCL-61), wobei Calciumphosphat-Fällung verwendet wurde (J. Sambrook et al., Molecular Cloning; A laboratory manual, Cold Spring Laboratory Press, Cold Spring Harbor, U. S. A., 1989, S. 1637). CHO-K1-Zellen wurden etabliert, um LRP konstitutiv an ihrer Zelloberfläche zu exprimieren (D. J. FitzGerald et al., J. Cell. Biol., Bd. 129,1995, S. 1533-1541). Stabil exprimierende CHO K1-Zellen wurden unter Selektion mit G-148 (Gibco-BRL, Breda, Niederlande) mit einer Konzentration von 800 µg/ml erhalten.

CHO-K1-Zellen, die die leichte Kette von Faktor VIII stabil exprimierten, wurden in 2 Vertiefungen einer Platte mit 6 Vertiefungen (Nunc A/S, Roskilde, Dänemark) bis zur Konfluenz gezüchtet. Die Vertiefungen wurden fünf Mal unter Verwendung von Dulbeccos modifiziertem Eagle-Medium-F12-Medium (DMEM-F12) (Gibco, BRL, Breda, Niederlande) gewaschen, und 1 ml DMEM-F12 wurde zugegeben. In eine der Vertiefungen würde der LRP-Antagonist RAP bis zu einer Konzentration von 20 mg/ml sofort und 2 und 4 Stunden nach dem Waschen der Zellen zugegeben. Proben wurden bis zu sechs Stunden nach dem Waschen der Zellen entnommen und danach auf die Konzentration der leichten Kette von Faktor VIII analysiert, wobei ein auf diesem Gebiet bekanntes Verfahren verwendet wurde (Lenting P. J. et al., J. Biol. Chem., Bd. 269, 1994, S. 7150-7155). Wie in Fig. 3 gezeigt, nahm die Konzentration der leichten Kette von Faktor VIII im Medium im Laufe der Zeit in Abwesenheit von RAP zu. In Anwesenheit von RAP nimmt jedoch das Ausmaß des Anstiegs der leichten Kette von Faktor VIII, im Vergleich zur Abwesenheit von RAP, zu. Somit ist die Inhibition von LRP mit einer Akkumulation der leichten Kette von Faktor VIII im Medium verbunden. Dies zeigt deutlich, dass ein LRP abhängiger Mechanismus bei der Zell-Aufnahme der leichten Kette von Faktor VIII eine Rolle spielt.

### Beispiel IV : Wechselwirkung zwischen Faktor VIII und Zell-Oberflächen-exponiertem Low Density Lipoprotein-Rezeptor-verwandtem Protein

Wie in Beispiel III beschrieben, besteht eine Wechselwirkung zwischen der leichten Kette von Faktor VIII und dem Zell-Oberflächen-exponiertem LRP. Wir haben deshalb auch untersucht, ob das intakte Faktor VIII-Protein eine Wechselwirkung mit Zell Oberflächen-exponiertem LRP hat. Eine zuvor etablierte Mäuse Fibroblasten-Zellinie, die stabil transfiziert worden war, um Faktor VIII zu erzeugen (Mertens K. et al., Brit. J. Haematol., Bd. 85,1993,133-142) wurde in 2 Vertiefungen einer Platte mit 6 Vertiefungen bis zur Konfluenz gezüchtet (Nunc A/S, Roskilde, Dänemark). Die Zellen wurden fünf Mal unter Verwendung von Iscovs modifiziertem Eagle-Medium (IMEM) (Boehringer Ingelheim/ Biowhitaker, Verviers, Belgien) gewaschen, und 1 ml IMEM wurde zugegeben. In eine der Vertiefungen wurde der LRP-Antagonist RAP bis zu einer Konzentration von 20 mg/ml sofort und 2 und 4 Stunden nach dem Waschen der Zellen zugegeben. Proben wurden bis zu sechs Stunden nach dem Waschen der Zellen entnommen und danach auf Faktor VIII-Cofaktor-Aktivität analysiert, wobei ein bereits etabliertes Verfahren verwendet wurde (Mertens K. et al., Brit. J. Haematol., Bd. 85,1993,133-142). Wie in Fig. 4 gezeigt, steigt die Menge der Faktor VIII-Cofaktor-Aktivität im Medium im Laufe der Zeit in Abwesenheit von RAP zu. In Anwesenheit von RAP nimmt jedoch das Ausmaß des Faktor VIII-Anstiegs im Vergleich zur Abwesenheit von RAP zu. Somit ist die inhibition von LRP mit einer Akkumulation von Faktor VIII im Medium verbunden. Es ist daher offensichtlich, dass ein LRP-abhängiger Mechanismus bei der Zell-Aufnahme der leichten Kette von Faktor VIII eine Rolle spielt.

### Beispiel V : Die Wirkung von RAP auf die Faktor VIII-Pharmakokinetik bei Knock-out-Mäusen mit schwerer Faktor VIII-Defizienz.

Auf gentechnischem Wege wurde ein Mäusestamm mit schwerer Faktor VIII (FVIII)-Defizienz durch gezielte Disruption des Mäuse-Faktor VIII-Gens gemäß Bi et al., Nature Genetics, 1995, Bd. 10, S. 119-121, geschaffen. Faktor VIII-Knock-out-Mäuse wurden durch eine Insertion eines neo-Gens in das 3'-Ende von Exon 17 des Mäuse-Faktor VIII-Gens geschaffen. Die betroffenen männlichen Tiere (X'Y) hatten nicht nachweisbare Faktor VIII-Levels von < 0,02 0,01 E/ml, wenn Messungen entweder mit einem chromogenen Faktor VIII-Test, Hyland Immuno, Wien, Österreich, wie kürzlich beschrieben (Turecek et al., Thromb. Haemostas. Suppl., 1997, Bd. 769) oder mittels Antigen-ELSSA, wie nachstehend beschrieben, vorgenommen wurden.

Zwei betroffene, hemizygote männliche Mäuse (X'Y) wurden intravenös mit einer Dosis von 200 E/kg Körpergewicht einer rekombinanten Human-Faktor VIII (rhFVIII)-Präparation behandelt, die von Chinese Hamster-Ovarien-Zellen stammte, die wie beschrieben hergestellt worden waren (WO/85/01961), und ohne stabilisierendes Protein pharmazeutisch formuliert worden war.

Unter Narkose wurden eine Stunde nach der Behandlung die Schwanzspitzen der Mäuse mit der Klinge eines Skalpells, wie von Novak et al., Brit. J. Haematol. Bd. 69,1998, S. 371-378 beschrieben, eingeschnitten. Ein Volumen von 50 µl Blut wurde mit Kapillarröhrchen (Ringcaps, Hirschmann, Deutschland) aus den Schwanzwunden gesammelt, wobei die Kapillarröhrchen mit Lithium Heparin als Antikoagulans beschichtet waren. Die Kapillarröhrchen wurden geschlossen und zentrifugiert, um die Blutzellen vom Plasma zu trennen. Danach wurden die Kapillarröhrchen geöffnet, und die Zell-und die Plasma-Fraktion wurden durch weiteres Zentrifugieren gesammelt. Schließlich wurden die Plasmaproben der Faktor VIII-Bestimmung mittels Faktor VIII-Antigen ELISA, Test Set IMMUNOZYM FVIII Ag, Hyland Immuno, Wien, Österreich, unter Verwendung von monoklonalen Antifaktor VIII-Antikörpern sowohl für das Abfangen wie für den Nachweis, wie beschrieben (Stel et al., Nature, 1983, Bd. 303, S. 530-532; Lenting et al., J. Biol. Chem., Bd. 269,1994, S. 7150-7155; Leyte et al., Biochem. J., Bd. 263,1989, S. 187-194) unterzogen. Die resultierenden Faktor VIII-Werte wurden in internationalen Einheiten von humanem Faktor VIII ausgedrückt. Die Ergebnisse der Faktor VIII-Plasma Level sind in der Tabelle angegeben.

Zwei andere betroffene hemizygote männliche Mäuse (X'Y) wurden mit rekombinantem Rezeptor-assoziiertem Protein (GST-RAP) 10 min vor der Behandlung mit dem rekombinanten humanen Faktor VIII mit einer Dosis von. 40 mg/kg Körpergewicht vorbehandelt. Das bei dieser Untersuchung verwendete RAP, welches mit LRP in Wechselwirkung tritt, wurde durch bakterielle Fermentation, wie von Hertz et al., (J. Biol. Chem., Bd. 266,1991, S. 21232-21238) beschrieben, erhalten. Ein Fusionsprotein des RAP mit Glutathion-S-Transferase wurde in E. coli exprimiert und mittels Affinitätschromatographie auf Glutathion-Agarose gereinigt. Das resultierende Protein bestand hauptsächlich aus dem Fusionsprotein und Spaltprodukten von RAP und Glutathion-S-Transferase. Das Fusionsprotein wurde in einem injizierbaren Puffer bereit für eine Verabreichung an die Faktor VIII-Knock-out-Mäuse formuliert. Wie bei der Kontrollgruppe (Behandlung nur mit Faktor VIII) wurden Blutproben eine Stunde nach der Verabreichung von rekombinantem Faktor VIII genommen und unter Verwendung von Faktor VIII-Antigen-ELISA auf Faktor VIII-Aktivität gemessen.

Die Ergebnisse sind in Tabelle III angeführt.

**Tabelle III**

| | Behandlung Dosis | Behandlung Dosis | Gewinnung 1 h nach Behandlung |
|---|---|---|---|
| Maus Nr. | GST-RAP | rhFVIII | FVIII:Ag (E/ml Plasma) |
| 1 | 40 mg/kg | 200 E/kg | 1,92 |
| 2 | 40 mg/kg | 200 E/kg | 1,88 |
| 3 | - | 200 E/kg | 0,73 |
| 4 | - | 200 E/kg | 0,83 |

Bei mit GST-RAP vorbehandelten Mäusen betrug der Faktor VIII Level mehr als 200 % der Plasma-Level nach der Behandlung mit rekombinantem Faktor VIII allein. Die Verabreichung des LRP Antagonisten, RAP, verbesserte die Pharmakokinetik von Faktor VIII.

### Beispiel VI : Identifizierung potentieller LRP-Bindungs-Exo Stellen auf der leichten Kette von Faktor VIII.

Ein Verfahren zur Identifizierung von Exo-Stellen, die möglicherweise bei der Protein-Protein-Wechselwirkung eine Rolle spielen, wurde bereits etabliert (J. Kyte und R. F. Doolittle, J. Mol. Biol. Bd. 157,1982, S. 105-132). Dieses Verfahren sieht ein Programm vor, welches die Hydrophilie und Hydrophobie eines Proteins entlang seiner Aminosäuresequenz fortlaufend evaluiert. Bei dem Verfahren wird eine Hydropathie-Skala verwendet, die die durchschnittliche Hydropathie innerhalb von Segmenten vorbestimmter Größe entlang der Aminosäuresequenz wiedergibt. Hydrophile Abschnitte sind durch negative Hydropathie-Werte charakterisiert und weisen wahrscheinlich eine Orientierung zur Außenseite eines Proteins auf, welches in einer wässerigen Lösung vorhanden ist. Dieses Verfahren wurde auf die bekannte Sequenz von Human-Faktor VIII angewandt (G. A. Vehar et al., Nature, Bd. 312,1984, S. 337-342; J. J. Toole et al., Nature, Bd. 312,1984, 342-347), wobei eine Segmentgröße ("Fenster") von 19 Resten verwendet wurde. Aus der vollständigen Sequenz des Faktors VIII wurde die Region 1690-2332, die der Faktor VIII A3/C1/C2-Domäne entspricht, dieser Analyse unterzogen, und der resultierende Hydropathie-Plot, der einen Wert von-15 als "cut off" hat, ist in den Fig. 5A und 5B gezeigt.

Dieses Verfahren zeigte mehrere getrennte Regionen mit niedrigen Hydropathie-Werten, die die hydrophile Natur, die mit potentiellen Exo-Stellen verbunden ist, widerspiegelt. Diese sind als A bis K angegeben (Tabelle IV) :

**Tabelle IV**

| Stelle | Reste | Domäne |
|---|---|---|
| A | Met 1711 bis Gly 1725 | A3 |
| B | Phe 1743 bis Arg 1749 | A3 |
| C | Ser 1784 bis Asp 1831 | A3 |
| D | Ser 1888 bis His 1919 | A3 |
| E | Trp 1942 bis Met 1947 | A3 |
| F | Ser 1959 bis Ala 1974 | A3 |
| G | Ile 2037 bis Trp 2062 | C1 |
| H | Asp 2108 bis Asn 2118 | C1 |
| I | Thr 2154 bis Ile 2158 | C1 |
| J | Arg 2209 bis Phe 2234 | C2 |
| K | His 2269 bis Lys 2281 | C2 |

**Tabelle V:**

| Stelle | Reste | Domäne |
|---|---|---|
| I | Phe 1785 bis His 1822 | A3 |
| II | Trp 1889 bis Asn 1915 | A3 |
| III | Trp 2112 bis Tyr 2115 | C1 |
| IV | His 2211 bis Leu 2230 | C2 |

Wiederum wurde aus der vollständigen Sequenz von Faktor VIII die Region 1690-2332, die der leichten Kette von vollständigem Faktor VIII entspricht, dieser Analyse unterzogen, und der resultierende Hydropathie-Plot, der einen Wert von-20 als "cut off" hat, ist in Fig. 5B gezeigt. Die Exo-Stellen sind als I bis IV angezeigt.

### Beispiel VII : Die C2-Domäne von Faktor VIII enthält eine LRP Bindungsstelle.

Die A3-C1-C2-Region des Faktors VIII umfasst den Bindungsanteil für LRP (vgl. Beispiel I). Diese Region enthält eine Anzahl potentieller LRP-Bindungs-Exo-Stellen in den sie bildenden Domänen, einschließlich der C2-Domäne (vgl. Beispiel VI). Zur Bestätigung, dass solche Exo-Stellen tatsächlich bei der LRP-Bindung eine Rolle spielen könnten, wurde die Wechselwirkung zwischen LRP und der C2-Domäne des Faktors VIII genauer analysiert. Die C2-Domäne des Faktors VIII (d. h. die Reste 2172-2332) wurde in Insektenzellen unter Verwendung einer bereits etablierten Methode (K. Fijnvandraat et al., Blood, Bd. 91,1998, S. 2347-2352) exprimiert. Die C2-Domäne des Faktors VIII wurde mittels Immunoaffinitätschromatographie unter Verwendung des auf die C2-Domäne gerichteten monoklonalen Antikörpers CLB-CAg 117 (K. Fijnvandraat et al., Blood, Bd. 91,1998, S. 2347-2352) gereinigt. Die Wechselwirkung mit LRP wurde unter Verwendung der Oberflächen-Plasmon-Resonanz-Analyse an einem BIAcoreTM2000-System (Pharmacia Biosensor AB, Uppsala, Schweden) untersucht. LRP wurde auf einem CM5-Sensor-Chip, wie in Beispiel I beschrieben, immobilisiert. Zur Verstärkung des Resonanz-Signals wurde die C2-Domäne des Faktors VIII (0,100 oder 375 nM) in Anwesenheit von 500 nM des auf die C2-Domäne gerichteten monoklonalen Antikörpers ESH-8 (D. Scandella et al., Blood, Bd. 86,1995, S. 1811-1819) in 50 mM HEPES (pH 7,4), 150 mM NaCl, 2 mM Cal₂,0,005% (V/V) Tween-20 15 Minuten lang bei Raumtemperatur vorinkubiert. Danach wurden vorinkubierte Proben über den Kontroll-Kanal geleitet, um die unspezifische Bindung zu bewerten, sowie aber den LRP-beschichteten Kanal (8,3 fmol/mm2), mit einer Strömung von 5 ml/min, 2 min lang, bei 25°C.

In Abwesenheit der C2-Domäne zeigt ESH-8-wenn überhaupt-eine minimale Bindung an immobilisiertes LRP. Jedoch in Anwesenheit der C2-Domäne wurde ein Dosis-abhängiger Anstieg der Bindung an LRP beobachtet (Fig. 6). Dies zeigt, dass die C2-Domäne des Faktors VIII an LRP bindet. Somit weisen die Exo-Stellen innerhalb der leichten Kette von Faktor VIII sehr wohl die Fähigkeit einer LRP-Bindung auf und somit die Fähigkeit, in der LRP-abhängigen Clearance von Faktor VIII in vivo eine Rolle zu spielen.

### Beispiel VIII : Bindung der FVIII C2-Domäne in Gegenwart eines anti-FVIII-C2-Domänen-Antikörpers

Das FVIII-Molekül umfasst zwei Stellen, die bei der vWF-Bindung involviert sind, wobei eine dieser Stellen an der Carboxy-terminalen C2-Domäne der leichten Kette von Faktor VIII liegt (Saenko und Scandella, J .B.C. 272 (1997),S. 18007-18014). Da die vWF-Bindung durch den auf die C2-Domäne gerichteten Antikörper ESH4 inhibiert wird, wurde dieser Effekt auf die LRP-Bindung untersucht, wobei die Antikörper-Bindungskörperuntersuchungen wie in Beispiel VII durchgeführt wurden. Der Antikörper ESH4 wurde von American Diagnostica erhalten.

Wie in Fig. 7 gezeigt, interferiert ESH4 mit der Bindung von LRP an die leichte Kette von Faktor VIII. Diese Inhibierung scheint spezifisch zu sein, da ESH4 nicht in der Lage war, die Bindung von tissue-type-Plasminogenaktivator/Plasminogenaktivator-Inhibitor-1-Komplexen an LRP zu beeinflussen. Weiters waren andere Antikörper, die gegen die leichte Kette von Faktor VIII gerichtet waren, nämlich CLB-CAg A und CLB-CAg 69 (Lenting et al., J.B.C. 269 (1994), S. 7150-7155) nicht in der Lage, mit LRP hinsichtlich der Bindung an Faktor VIII zu interferieren.

In Fig. 7 wird dabei die Bindung der Faktor VIII-C2 Domäne an LRP in Gegenwart von ESH4 gezeigt. Hierbei wurde immobilisiertes LRP (16 fmol/mm²) mit der leichten Kette von Faktor VIII (150 nM) in Gegenwart oder Abwesenheit von Antikörper ESH4 bei einem Fluss von 5µl/min für 2 min bei 25°C inkubiert. Die Ergebnisse wurden in Resonanzeinheiten (resonance units; RU) angegeben und gegen nicht spezifische Bindung korrigiert, die bei weniger als 5% gegenüber der Bindung an LRPgecoateten Kanälen lag.

In Fig. 7 wurde dabei die Konzentration des ESH4-Antikörpers in nM auf der x-Achse und die verbleibende Bindung der leichten Kette von Faktor VIII in RU auf der y-Achse aufgetragen.

### Beispiel IX : Die Faktor VIII A3-C1-Region weist eine LRP Bindungsstelle auf

Die A3-C1-C2-Region des Faktor VIII umfasst den Bindungsanteil für LRP (vgl. Beispiel I). Aus der in Beispiel II beschriebenen kinetischen Analyse geht klar hervor, dass mehrfach Stellen, die bei der LRP eine Rolle spielen, vorhanden sind (Tabelle II, Bindungsstellen der Klasse A und der Klasse B). Wie in Beispiel VII angedeutet, wurde das Vorliegen solcher interaktiven Stellen für die Region der Faktor VIII C2-Domäne bewiesen (vgl. auch Fig. 6). Zur Bestätigung, dass auch andere Exo-Stellen bei der Wechselwirkung mit LRP eine Rolle spielen, wurde die Wechselwirkung zwischen LRP und der Faktor VIII A3-C1-Region (d. h. Faktor VIII Reste 1649 bis 2172) analysiert. Um das Fragment der leichten Kette dieses Faktor VIII zu erhalten, wurde ein Konstrukt hergestellt, das für das Signalpeptid des Faktor VIII, das mit den Resten 1649 bis 2172 verschmolzen ist, codiert. Dieses Konstrukt wurde wie folgt hergestellt. Der Vektor pcFaktor VIII-LC, welcher in Beispiel III beschrieben ist, wurde als Konformationsschablone für die Konstruktion eines Faktor VIII-Fragments unter Verwendung von PCR verwendet. Dieses Fragment wurde unter Verwendung des sense-Primers A1 (5'-TTA GGA TCC ACC ACT ATG CAA ATA GAG CTC TCC-3', vgl. Beispiel III) und des antisense-Primers FA2172min (5'-AAT GCG GCC GCT TCA ATT TAA ATC ACA GCC CAT-3') erzeugt. Der Primer FA 2172 codiert für eine Notl-Erkennungsstelle, ein Stopcodon und die Reste 2167 bis 2172 des Faktor VIII. Das PCR-Produkt wurde mit BspMII und Notl gespalten, und ein 352 Basenpaar-Fragment wurde isoliert und in den pcFaktor VIII-LC-Vektor ligiert, der unter Verwendung derselben Restriktionsenzyme verdaut wurde. Der resultierende Vektor, der als pcFaktor VIII-A3C1 bezeichnet wurde, wurde auf CHO-K1-Zellen (ATCC CCL-61) unter Verwendung von Kalziumphosphatfällung transfiziert (J. Sambrook et al., Molecular Cloning ; A laborary manual, Cold Spring Laboratory Press, Cold Spring Harbor, U. S. A., 1989, S. 1637)-CHO-K1-Zellen, die Faktor VIII A3-C1 Fragmente stabil exprimieren, wurden unter Selektion mit G-148 (Gibco-BRL, Breda, Niederlande) in einer Konzentration von 800 µg/ml erhalten.

CHO-K1-Zellen, die Faktor VIII A3-C1-Fragmente stabil exprimieren, wurden für die Kultivierung in großem Umfang verwendet, um konditioniertes Medium zu erhalten, das Faktor VIII A3-C1 enthält. Faktor VIII A3-C1 wurde mittels Immunaffinitätschromatographie gereinigt, wobei der zuvor beschriebene monoklonale Antikörper CLB-CAg A (Leyte A. et al., Biochem. J., 1989, Bd. 263, S. 187-194) verwendet wurde, welcher gegen die A3-Domäne des Faktor VIII gerichtet ist. Zu diesem Zweck wurde CLB-CAg A an CNBr-Sepharose 4B (Pharmacia Biotech, Roosendaal, Niederlande) gemäß den Instruktionen des Herstellers in einer Konzentration von 1 mg/ml immobilisiert. Konditioniertes Medium wurde mit CLB-CAg A-Sepharose (2 ml pro Liter Medium) inkubiert, und gebundener Faktor VIII A3-C1 wurde in 150 mM NaCl, 55 (V/V) % Ethylenglykol, 25 mM Lysin (pH 11) eluiert. Fraktionen, die Faktor VIII A3-C1 enthielten, wurden sofort unter Verwendung von 1/10 Volumen von 1 M Imidazol (pH 5,0) neutralisiert und danach gegen 150 mM NaCl, 2 mM CaCl₂, 0,005 % (V/V) Tween-20,20 mM HEPES (pH 7,4) dialysiert.

Die Wechselwirkung zwischen Faktor VIII A3-C1 und LRP wurde unter Verwendung der Oberflächen-Plasmon-Resonanz-Analyse an einem BIAcoreTM2000-System (Pharmacia Biosensor AB, Uppsala, Schweden) untersucht. LRP wurde auf einem CM5-Sensor-Chip, wie in Beispiel I beschrieben, immobilisiert. Proben, die Faktor VIII A3-C1 (200 nM oder 400 nM) enthielten, wurden mit einer Strömung von 5 µl/min 2 min lang bei 25°C aber den Kontrollkanal geleitet, um die nichtspezifische Bindung zu beurteilen und über den mit LRP überzogenen Kanal (8,3 fmol/mm2). In Tabelle VI ist der maximale Anstieg der Resonanz-Einheiten für beide Konzentrationen des Faktor VIII A3-C1 zusammengefasst. In Anwesenheit von 400 nM Faktor VIII A3-C1 wurde eine höhere Reaktion beobachtet als bei 200 nM Faktor VIII A3-C1 (59 bzw. 47 Resonanz-Einheiten). Um die Bindung von Faktor VIII A3-C1 an LRP zu verbessern, wurde Faktor VIII A3-C1 (400 nM) in Anwesenheit von 500 nM des monoklonalen Antikörpers CLB-CAg A in 50 mM HEPES (pH 7,4), 150 mM NaCl, 2 mM CaCl2,0,005 W (V/V) Tween 20 15 min lang bei Raumtemperatur vorinkubiert. Danach wurden die vorinkubierten Proben über den Kontrollkanal und über den mit LRP überzogenen Kanal mit einer Strömung von 5 ml/min 2 min lang bei 25°C geleitet. In Anwesenheit des Antikörpers CLB-CAg A wurde tatsächlich eine Erhöhung der Reaktion beobachtet (118 Resonanz-Einheiten). Somit zeigen diese Daten deutlich, dass Faktor VIII A3-C1 mit LRP in dosisabhängiger Weise in Wechselwirkung treten kann.

**Tabelle VI : Bindung von Faktor VIII A3-C1 an immobilisiertes LRP, wie unter Verwendung der SPR-Analyse nachgewiesen. Die Bindung an LRP ist in Resonanz-Einheiten ausgedrückt und ist auf unspezifische Bindung korrigiert.**

| Konzentration A3-C1 | Bindung (Resonanz-Einheiten) |
|---|---|
| 200 nM | 47 |
| 400 nM | 59 |
| 400 nM + 500 nM CLB-CAg A | 118 |

### Beispiel X : Wechselwirkung zwischen Faktor VIII A3-C1 und an der Zellenoberfläche exponiertem Low Density Lipoprotein-Receptorrelated-Protein

Wie oben beschrieben, kann Faktor VIII A3-C1 (d. h. die Reste 1649 bis 2172) mit gereinigtem LRP in Wechselwirkung treten. Wir haben weiters die Wechselwirkung zwischen Faktor VIII A3-C1 und LRP, der an Oberfläche lebender Zellen exprimiert wird, untersucht. Daher wurden CHO-K1-Zellen, die Faktor VIII A3-C1 stabil exprimieren (wie oben beschrieben), bis zur Konfluenz in 6 Vertiefungen einer Platte mit 24 Vertiefungen gezüchtet (Nunc A/S, Roskilde, Dänemark). Die Vertiefungen wurden fünfmal unter Verwendung von DMEM-F12 (Gibco-BRL, Breda, Niederlande) gewaschen, und 500 µl DMEM-F12 wurden zugegeben. In drei der Vertiefungen wurde der LRP-Antagonist RAP in einer Konzentration von 1 AM zum Zeitpunkt 2 und 4 Stunden nach dem Waschen der Zellen zugegeben. Proben wurden bis zu 6 Stunden nach dem Waschen der Zellen gezogen und danach auf die Konzentration von Faktor VIII A3-C1 analysiert. Die Konzentrationen von Faktor VIII A3-C1 wurden im wesentlichen unter Verwendung eines auf diesem Gebiet beschriebenen Verfahrens (Lenting P. J. et al., J. Biol. Chem., Bd. 269,1994, S. 7150-7155) bestimmt, außer, dass der monoklonale Antikörper CLB-CAg 12 anstelle von CLB-CAg 117 verwendet wurde. Wie in Fig. 8 gezeigt, nahm die Konzentration von Faktor VIII A3-C1 im Verlauf der Zeit in Abwesenheit von RAP zu. In Anwesenheit von RAP jedoch nimmt das Ausmaß von Faktor VIII A3-C1 im Vergleich zur Abwesenheit von RAP zu. Somit ist die Inhibition von RAP mit einer Ansammlung von Faktor VIII A3-C1 im Medium verbunden. Dies zeigt, dass bei der Aufnahme von Faktor VIII A3-C1 in die Zelle ein LRP-abhängiger Mechanismus eine Rolle spielt.

Fig. 8 zeigt die Wirkung des LRP-Antagonisten RAP auf die Konzentration von Faktor VIII A3-C1 in einem Medium von Faktor VIII A3-C1 exprimierenden Zellen. In Abwesenheit von RAP (offene Symbole) ist der Anstieg der Faktor VIII A3-C1-Mengen geringer als in Anwesenheit (geschlossene Symbole). Die-Daten repräsentieren den Mittelwert Standardabweichung der drei Versuche.

### Beispiel XI : Mutationen in der Faktor VIII-C2-Domäne beeinflussen die Bindung an LRP (beschreibt technischen Hintergrund)

Wie in Beispiel VII beschrieben, weist die Faktor VIII-C2-Domäne eine Bindungsstelle für LRP auf. Wir haben deshalb die Auswirkung von Mutationen in dieser Domäne auf die Bindung von Faktor VIII bzw. Wechselwirkung mit LRP, der an der Oberfläche der Zelle exponiert ist, untersucht. Um Faktor VIII-Varianten, die solche Mutationen aufweisen, zu exprimieren, wurden zwei Konstrukte hergestellt. Beide Faktor VIII-Expressionsplasmide waren Derivate des Plasmids pF8-SQ#428 (F. Scheiflinger, unveröffentlichte Resultate) ; ein Plasmid, das die cDNA einer B-Domänen-deletierten FVIII-Variante in den im Handel erhältlichen Vektor pSI (Promega) insertiert enthält. Bei diesem Konstrukt wurden alle außer vierzehn Aminosäuren der B-Domäne von FVIII entfernt (SQ Mutante, vgl. Lind et al., Eur. J. Biochem., Bd. 232,1995, S. 19-27).

Zuerst erfolgte eine Modifikation des Vektors pF8-SQ#428, indem der Vektor mit EcoRV/Agel geschnitten wurde ; und Ligation der angelagerten Oligonukleotide P-A/Em (1) 5'-CCGGAGATTA TTACGAGGAC AGTTATGAAG AC-3'und P-A/Em (2) 5'-GTCTTCATAA CTGTCCTCGT AATAATCT-3'. Diese Vorgangsweise ergab den Vektor pF8-SQdA/E#501. Innerhalb dieses Vektors wird die Expression der FVIII-cDNA durch den SV40-Promoter und Enhancer angetrieben. Stromabwärts, am 3'-Ende des FVIII-Gens, wird die Polyadenylierungsstelle von SV40 verwendet, um die Transkription zu beenden. Ein chimäres Intron, zusammengesetzt aus der 5'-Donor-Stelle aus dem ersten Intron des humanen ss-Globin-Gens und dem Zweig und der 3'-Akzeptor-Stelle aus dem Intron, das sich zwischen dem Leader und dem Körper einer variablen Region einer schweren Kette eines Immunglobulin-Gens befindet, wurde eingeführt, um die Menge der Genexpression zu erhöhen (vgl. pSI, Produktinformation, Promega). Um die Expressionsmengen weiter zu verbessern, wurde ein Sequenz-Kontext, der sich für die Initiierung der eukaryontischen Protein-Translation als optimal erwies ("Kozak Sequenz" 5'-GCCACCATG-3'), unmittelbar stromaufwärts des FVIII Start-Codons (Kozak, J. Biol. Chem., Bd. 266,1991, S. 1986719870) insertiert. Der Vektor pF8-SQ-dA/E#501 wurde dann zur Konstruktion der Vektoren pC2-m7#516 und pC2-m9#518 verwendet.

Die Konstruktion von pC2-m7#516 erfolgte durch Insertion der angelagerten Oligonukleotide Mu (1) 5'-CGAATTCACC CCCAGATTTG GGAACACCAG ATTGCCCTGA GGCTGGAGAT TCTGGGCTGC GAGGCACAGC AGCAGTACTG AGC-3' und P-Mu (2) 5'-GGCCGCTCAG TACTGCTGCT GTGCCTCGCA GCCCAGAATC TCCAGCCTCA GGGCAATCTG GTGTTCCCAA ATCTGGGGGT GAATT-3' n den mit Asull/Notl-geschnittenen Vektor pF8-SQ-dA/E#501.

Der resultierende Vektor codiert für eine Faktor VIII-Variante, die die folgenden Substitutionen aufweist: Ser²³¹² bis Ile²³¹², Val²³¹⁴ bis Glu²³¹⁴, Met²³²¹ bis Leu²³²¹, Val²³²³ bis Ile²³²³, Asp²³³⁰ bis Gln²³³⁰ und Leu²³³¹ bis Gln²³³¹.

Die Konstruktion von pC2-m9#518 erfolgte durch Insertion der angelagerten Oligonucleotide P-CC (1) 5'-CTAGAACCAC CGTTAGTGGC TCGCTACGTG CGACTGCACC CCCAGAGTTG GGCTCACCAT-3', P-CC (2) 5' ATTGCCCTGA GGCTGGAGGT TCTGGGCTGC GATACTCAGC AGCCAGCTTG AGC-3', P-CC (3) 5'-GGCCGCTCAA GCTGGCTGCT GAGTATCGCA GC-3', P-CC (4) 5' CCAGAACCTC CAGCCTCAGG GCAATATGGT GAGCCCAACT CTGGGGGTGC-3' und PCC (5) 5'-AGTCGCACGT AGCGAGCCAC TAACGGTGGT T-3' in den mit Xbal/Notl geschnittenen Vektor pF8-SQ-dA/E#501.

Das Konstrukt pC2-m9#518 kodiert für eine Faktor VIII-Variante, die die folgenden Aminosäuresubstitutionen umfasst : Asp²²⁹⁸ bis Glu²²⁹⁸, Leu²³⁰² bis Val²³⁰², Thr²³⁰³ bis Ala²³⁰³ und Leu²³⁰⁶ bis Val²³⁰⁶, Ile²³⁰⁸ bis Leu²³⁰⁸, Val²³¹⁴ bis Ala²³¹⁴, Gln²³¹⁶ bis His²³¹⁶, Met²³²¹ bis Leu²³²¹, Glu²³²⁷ bis Asp²³²⁷, Ala²³²⁸ bis Thr²³²⁸, Asp²³³⁰ bis Gln²³³⁰, Leu²³³¹ bis Pro²³³¹ und bis Tyr²³³² bis Ala²³³².

Beide Vektoren, pC2-m9#518 und pC2-m7#516, die für Faktor VIII#518 bzw. Faktor VIII#516 kodieren, wurden in Maus-Fibroblasten-C127-Zellen transfiziert, wobei die Calciumphosphatfällung verwendet wurde (J. Sambrook et al., Molecular Cloning; A laboratory manual, Cold Spring Laboratory Press, Cold Spring Harbor, U. S. A., 1989, S. 1637). Die Vektoren wurden ko-transfiziert (Verhältnis 20 : 1), wobei das Plasmid pDH#310 verwendet wurde, was eine Selektion der Transfektanten mit Hygromycin B (200g/ml) ermöglichte.

C127-Zellen, die normalen Faktor VIII (vgl. Beispiel IV), Faktor VIII#518 und Faktor VIII#516 stabil exprimieren, wurden zu 50% Konfluenz in 4 Vertiefungen einer Platte mit 24 Vertiefungen gezüchtet (Nunc A/S, Roskilde, Dänemark).
Die Vertiefungen wurden fünf Mal unter Verwendung von IMEM (Boehringer Ingelheim/ BioWhitaker, Verviers, Belgien) gewaschen, und 1 ml IMEM wurde zugesetzt. In zwei der Vertiefungen für jede Faktor VIII-Varian te wurde der LRP-Antagonist RAP bis zu einer Konzentration von 20 mg/ml sofort und 2 h nach dem Waschen der Zellen zugegeben. Proben wurden zwei und drei Stunden nach dem Waschen der Zellen genommen und danach auf Faktor VIII-Cofaktor-Aktivität unter Verwendung eines bereits etablierten Verfahrens analysiert (Mertens K. et al., Brit. J. Haematol., Bd. 85,1993,133-142). In der Tabelle VII sind die Faktor VIII-Expressionsmengen in Anwesenheit und Abwesenheit von RAP zu verschiedenen Zeitpunkten angegeben. Für normalen Faktor VIII sowie für die Faktor VIII Varianten #516 und #518 wird ein Anstieg der Faktor VIII-Aktivität im Medium in Anwesenheit oder Abwesenheit von RAP beobachtet. Für normalen Faktor VIII jedoch ist die Expressionsmenge in Anwesenheit von RAP erhöht, wogegen diese Wirkung für die Faktor VIII-Varianten #516 und #518 stark reduziert ist, sofern sie überhaupt vorhanden ist. Dies zeigt, dass die LRP-vermittelte Zell-Aufnahme der Faktor VIII-Varianten #516 und #518 weniger effizient verläuft als bei normalem Faktor VIII. Somit machen die Aminosäuresubstitutionen in der Faktor VIII-C2-Domäne, wie sie für die Faktor VIII-Varianten #516 und #518 angezeigt sind, den Faktor VIII weniger sensitiv für eine durch LRP vermittelte Zell-Aufnahme.

**Tabelle VII : Expressionsmengen von normalem Faktor VIII und den Faktor VIII-Varianten #516 und #518 in Anwesenheit und Abwesenheit von RAP. Die Daten repräsentieren den ±Mittelwert von zwei unabhängigen Versuchen.**

| Faktor VIII | Zeit (h) | Expression (E/L) | | Verhältnis |
|---|---|---|---|---|
| | | -RAP | +RAP | +RAP/-RAP |
| normal | 2 | 1,3 ± 0,1 | 4,2 ± 0,4 | 3,2 |
| | 3 | 2,2 ± 0,2 | 5,0 ± 0,3 | 2,3 |
| | | | | |
| #516 | 2 | 0,3 ± 0,2 | 0,3 ± 0,2 | 1,0 |
| | 3 | 0,6 ± 0,1 | 0,8 ± 0,3 | 1,3 |
| | | | | |
| #518 | 2 | 1,1 ± 0,1 | 1,3 ± 0,4 | 1,2 |
| | 3 | 3,3 ± 0,2 | 3,5 ± 0,3 | 1,1 |

## Patentansprüche

1. Humanes Faktor VIII-Polypeptid mit Faktor VIII:C-Aktivität, welches eine Modifikation zwischen, AS 1784 (Ser) und 1831 (Asp), und/oder AS 2108 (Asp) und 2118 (Asn) aufweist, wobei die Modifikation eine Mutation, Deletion oder Insertion ist, die die Bindungsaffinität zu dem Low Density Lipoprotein-Receptorverwandtem Protein (LRP) verringert.

2. Faktor VIII-Polypeptid nach Anspruch 1, **dadurch gekennzeichnet, dass** die Modifikation zwischen, AS 2108 (Asp) und 2118 (Asn) vorliegt.

3. Faktor VIII-Polypeptid nach Anspruch 2, **dadurch gekennzeichnet, dass** die Modifikation zwischen AS 2112 (Trp) und 2115 (Tyr) vorliegt.

4. DNA-Molekül, welches für ein Faktor VIII-Polypeptid mit Faktor VIII:C-Aktivität nach einem der Ansprüche 1 bis 3 codiert.

5. Expressionsvektor, welcher ein DNA-Molekül nach Anspruch 4 aufweist.

6. Transformierte Zelle und Nachkommenschaft derselben, welche ein DNA-Molekül nach Anspruch 4 oder einen Expressionsvektor nach Anspruch 5 aufweist.

7. Verfahren zur Herstellung eines Faktor VIII-Polypeptids mit Faktor VIII:C-Aktivität nach einem der Ansprüche 1 bis 3, welches die folgenden Schritte umfasst:
• Züchten einer Wirtszelle in einem Nährmedium, wobei die Wirtszelle einen Expressionsvektor aufweist, der in Transkriptionsrichtung eine Transkriptions-Regulierungsregion und eine Translations-Initiierungsregion enthält, die in einer Wirtszelle wirkt,
eine DNA-Sequenz, die für ein Polypeptid, wie in einem der Ansprüche 1 bis 3 definiert, codiert, und Translatlons- und Transkriptions-Terminations-Regionen, die in der Wirtszelle wirken, wobei die Expression der DNA-Sequenz durch die Initiierungs- und Terminations-Regionen reguliert wird, und
• Isolieren des Polypeptids.

8. Präparation, welche ein FVIII-Molekül mit FVIII:C-Aktivität nach einem der Ansprüche 1 bis 3 und ein Polypeptid ausgewählt aus der Gruppe der LRP-Antagonisten aufweist.

9. Präparation nach Anspruch 8, **dadurch gekennzeichnet, dass** der LRP-Antagonist ausgewählt ist aus der Gruppe von RAP und löslichen Fragmenten von LRP, die eine Bindungs-affinität zur FVIII-LRP-Bindungsstelle zeigen.

10. Verwendung eines Faktor VIII-Polypeptids nach einem der Ansprüche 1 bis 3 oder einer Präparation nach einem der Ansprüche 8 oder 9 zur Formulierung einer Präparation zur Behandlung einer Gerinnungsstörung.

11. Verwendung einer Präparation nach Anspruch 10, wobei die Gerinnungsstörung Hämophilie A ist.

## Claims

1. Human factor VIII-polypeptide which has factor VIII:C-activity comprising a modification between aa 1784 (Ser) and 1831 (Asp), and/or aa 2108 (Asp) and 2118 (Asn), wherein the modification is a mutation, deletion or insertion which decreases the binding affinity to the low density lipoprotein-receptor-related protein (LRP).

2. Factor VIII-polypeptide according to claim 1, wherein the modification is located between aa 2108 (Asp) and 2118 (Asn).

3. Factor VIII-polypeptide according to claim 2, wherein the modification is located between aa 2112 (Trp) and 2115 (Tyr).

4. DNA molecule encoding a factor VIII-polypeptide according to any of claims 1 to 3, which has factor VIII:C-activity.

5. Expression vector comprising a DNA-molecule according to claim 4.

6. Transformed cell and progeny thereof comprising a DNA-molecule according to claim 4 or an expression vector according to claim 5.

7. Method for preparation of a factor VIII-polypeptide which has factor VIII:C-activity according to any of claims 1 to 3, comprising the following steps of:
• growing a host cell in a culture medium, wherein said host cell comprises an expression vector which, in the direction of transcription, contains a transcription regulation region and a translation initiation region which is effective in a host cell,
a DNA sequence encoding a polypeptide according to any of claims 1 to 3, and translation- and transcription-termination regions which are effective in said host cell wherein the expression of said DNA sequence is regulated by said initiation and termination regions, and
• isolating the polypeptide.

8. Preparation comprising a FVIII molecule according to any of claims 1 to 3, which has FVIII:C-activity and a polypeptide selected from the group of LRP antagonists.

9. Preparation according to claim 8, wherein the LRP antagonist is selected from the group of RAP and soluble fragments of LRP which exhibit a binding affinity to the FVIII-LRP-binding site.

10. Use of a factor VIII-polypeptide according to any of claims 1 to 3 or of a preparation according to any of claims 8 or 9 for the formulation of a preparation for the treatment of a coagulation defect.

11. Use of a preparation according to claim 10, wherein the coagulation defect is hemophilia A.

## Revendications

1. Polypeptide facteur VIII humain à activité de facteur VIII:C, qui comporte une modification entre AA 1784 (Ser) et 1831 (Asp), et/ou AA 2108 (Asp) et 2118 (Asn), où la modification est une mutation, une délétion ou une insertion qui réduit l'affinité de liaison avec la protéine apparentée au Low Density Lipoprotein-Receptor (LRP).

2. Polypeptide facteur VIII selon la revendication 1, **caractérisé en ce que** la modification est située entre AA 2108 (Asp) et 2118 (Asn).

3. Polypeptide facteur VIII selon la revendication 2, **caractérisé en ce que** la modification est située entre AA 2112 (Trp) et 2115 (Tyr).

4. Molécule d'ADN qui code un polypeptide facteur VIII à activité de facteur VIII:C selon l'une des revendications 1 à 3.

5. Vecteur d'expression qui comporte une molécule d'ADN selon la revendication 4.

6. Cellule transformée et sa descendance, qui comportent une molécule d'ADN selon la revendication 4 ou un vecteur d'expression selon la revendication 5.

7. Procédé pour produire un polypeptide facteur VIII à activité de facteur VIII:C selon l'une des revendications 1 à 3 qui comprend les étapes suivantes :
• cultiver une cellule hôte dans un milieu nutritif, où la cellule hôte comporte un vecteur d'expression qui, dans la direction de la transcription, contient une région de régulation de la transcription et une région d'initiation de la traduction, qui agit dans une cellule hôte,
une séquence d'ADN qui code un polypeptide selon l'une des revendications 1 à 3, et
des régions de terminaison de la traduction et de la transcription, qui agissent dans ladite cellule hôte, où l'expression de ladite séquence d'ADN est régulée par lesdites régions d'initiation et de terminaison,
et
• isoler le polypeptide.

8. Préparation qui comporte une molécule de FVIII à activité de FVIII:C selon l'une des revendications 1 à 3 et un polypeptide choisi dans le groupe des antagonistes de LRP.

9. Préparation selon la revendication 8, **caractérisée en ce que** l'antagoniste de LRP est choisi dans le groupe de RAP et des fragments solubles de LRP qui présentent une affinité de liaison pour le site de liaison de LRP de FVIII.

10. Utilisation d'un polypeptide facteur VIII selon l'une des revendications 1 à 3 ou d'une préparation selon l'une des revendications 8 ou 9 pour la formulation d'une préparation pour le traitement d'un trouble de la coagulation.

11. Utilisation d'une préparation selon la revendication 10 où le trouble de la coagulation est l'hémophilie A.
